# EUROPEAN PATENT APPLICATION

(11) **EP 4 527 426 A1**
(43) Date of publication of application: **26.03.2025**
(21) Application number: 24201988.3
(22) Date of filing: 23.09.2024
(51) Int. Cl.: A61M 3/02

(54) **MULTIFUNCTIONAL IRRIGATION DEVICES**

(30) Priority: 21.09.2023 US 202363584468 P
(71) Applicant: Schultz, Joseph, P., Atlanta, GA 30342 (US)
(72) Inventor: Schultz, Joseph, P., Atlanta, GA 30342 (US)
(74) Representative: Bohest AG

(57) **Abstract**

Methods and devices for wound irrigation, abscess irrigation, ocular irrigation, and ear irrigation are disclosed with particular utility to address medical emergencies. Devices disclosed herein can comprise a particular arrangement of first and second nozzle that allow efficient conversion of the device as being suitable for one purpose to another.

## Description

### REFERENCE TO RELATED APPLICATION

This application claims the benefit of U.S. Provisional Patent Application No. 63/584,468, filed on September 21, 2023, the disclosure of which is incorporated herein by reference in its entirety.

### BACKGROUND

Irrigation systems are commonly employed in healthcare settings to safely and efficiently introduce water and solutions to an area of need for a patient.

U.S. 8,672,904 hereby incorporated by reference in its entirety, discloses splash shield systems for irrigation and lavage. The disclosure of the '904 patent includes several features that introduce an indirect stream of irrigation fluid from a fluid source within a shield to conduct the irrigation procedure.

U.S. 8,747,372 discloses various wound and abscess irrigation systems configured to introduce a direct stream of irrigation fluid from a fluid source within a shield to conduct the irrigation procedure, generally applied to the irrigation of wounds and abscesses.

As shown in these previous disclosures, irrigation systems each may have specific requirements tailored to their particular application. However, the requirements of irrigation procedures often may contradict one another, such that independent devices are commonly required to obtain the necessary effect depending on the intended irrigation procedure.

Particularly, irrigation procedures can require either of an indirect stream and a direct stream of fluid applied within a tissue or a surrounding, or a volume surrounding the tissue. Yet, specialized irrigation equipment and systems ultimately result in the need for several independent devices where a singular device incorporating the necessary features to conduct multiple irrigation procedures may not be available. This degree of specialization drives inefficiency which carries significant detriment both to administrative and organizational outcomes, as well as health outcomes in the emergency setting.

Accordingly, irrigation devices adaptable to perform multifunctional irrigation procedures which rely on direct and indirect streams of irrigation fluid are needed. It is to these multifunctional irrigation devices and methods of their use to which this disclosure is directed.

### SUMMARY

Multifunctional irrigation devices and methods of use are disclosed herein. In certain aspects, the multifunctional irrigation devices can comprise (i) a fluid source connector configured to connect to an irrigation fluid source, (ii) a first nozzle comprising a conduit configured to deliver irrigation fluid from the irrigation fluid source through a partition of the fluid source connector and into an irrigation end of the connector, (iii) optionally, a second nozzle removably secured to the first nozzle (e.g., via male/female threads, pressure fitting, clips, or prongs), and (iv) a shield configured to be removably attached to the fluid source connector (e.g., via male/female threads, pressure fitting, clips, or prongs).

For instance, in a particular aspect, multifunctional irrigation devices can comprise (i) a fluid source connector comprising a threaded inner surface and a partition, (ii) a first nozzle comprising a first nozzle inlet within the fluid source connector and a conduit extending across the partition to a first nozzle outlet outside of the fluid source connector, (iii) a second nozzle removably attached to the first nozzle to extend the conduit from the first nozzle outlet to a second nozzle outlet, (iv) a shield connector; and (v) a shield removably attached to the shield connector.

Methods of using the multifunctional devices are also disclosed and provide improvements to efficiency and utility over prior irrigation devices as noted by cross-reference above. In certain aspects, multifunctional devices disclosed herein can be used in a wound irrigation method and can comprise, or consist of, (i) attaching any one of the irrigation devices disclosed herein to a fluid source, (ii) positioning the shield in contact with the skin such that the first nozzle is directed toward a wound, and (iii) flowing an irrigation fluid through the irrigation device and toward the wound.

In other aspects, multifunctional devices disclosed herein can be used in an abscess irrigation method and can comprise, or consist of, (i) removing the shield from the irrigation device, (ii) positioning the first nozzle within the abscess, (iii) flowing an irrigation fluid through the irrigation device and into the abscess.

In other aspects, multifunctional devices disclosed herein can be used in an ocular irrigation method and can comprise, or consist of, (i) attaching any one of the irrigation devices disclosed herein to a fluid source, (ii) removing the first nozzle from the irrigation device, (iii) positioning the shield surrounding the eye, (iii) flowing an irrigation fluid through the irrigation device and into a cavity formed between the shield and the eye.

In other aspects, multifunctional devices disclosed herein can be used in an ear irrigation method and can comprise, or consist of (i) attaching any one of the irrigation devices disclosed herein to a fluid source, (ii) positioning the shield surrounding an ear, (iii) flowing irrigation fluid through the irrigation device and into the ear.

### BRIEF DESCRIPTION OF FIGURES

Figure 1 shows a prior art wound irrigation device.
Figure 2 shows a prior art abscess irrigation device.
Figure 3 shows a prior art wound and abscess irrigation system comprising a removable extension shield.
Figure 4 shows a further prior art wound irrigation device comprising multiple direct stream fluid outlets.
Figure 5 shows a prior art eye irrigation device.
Figure 6 shows a first inventive embodiment of a multifunctional irrigation device comprising a direct stream nozzle outlet and an indirect stream nozzle outlet.
Figure 7 shows a second inventive embodiment of a multifunctional irrigation device comprising a direct stream nozzle outlet and an indirect stream nozzle outlet.
Figure 8 shows a further inventive embodiment of a multifunctional irrigation system comprising interchangeable shields.
Figure 9 shows a further inventive embodiment of a multifunctional irrigation device comprising a concentric arrangement of indirect stream nozzle outlets.
Figure 10 shows a further inventive embodiment of a multifunctional irrigation system comprising a second nozzle removably attached to a first nozzle.
Figure 11 shows a further inventive embodiment of the multifunctional irrigation system shown in Figure 10.
Figure 12 shows a further inventive embodiment of a multifunctional irrigation system comprising a second nozzle removably attached to a first nozzle, the second nozzle comprising a direct stream nozzle outlet.
Figure 13 shows a further inventive embodiment of a multifunctional irrigation system comprising a second nozzle comprising an indirect stream nozzle outlet for abscess irrigation.
Figure 14 shows a further inventive embodiment of a multifunctional irrigation system configured for wound, abscess, and eye irrigation.
Figure 15 shows further inventive embodiments of a multifunctional irrigation system configured for wound, abscess, and eye irrigation.
Figure 16 shows further inventive embodiments of a multifunctional irrigation system configured for wound, abscess, and eye irrigation.
Figure 17 shows further inventive embodiments of a multifunctional irrigation system.
Figure 18 shows a multifunctional irrigation system analogous to that of Figure 17, assembled with a fixed saddle rim within the shield.
Figure 19 shows the multifunctional irrigation system of Figure 18 and each of its individual components and configurations.
Figure 20 shows a multifunctional irrigation system with a recessed first nozzle indirect stream outlet.
Figure 21 shows the multifunctional irrigation system of Figure 20 comprising a fixed saddle rim.
Figure 22 shows the multifunctional irrigation system of Figure 21 and each of its individual components and configurations.
Figure 23 shows a removable nozzle configured for use as an abscess drain.
Figure 24 shows a schematic of attachment of a multifunctional irrigation device comprising a seal opener to an irrigation fluid source.
Figure 25 shows a schematic of attachment of a second embodiment of multifunctional irrigation device comprising a seal opener to an irrigation fluid source.
Figure 26 shows a cross-section of a further inventive embodiment of a multifunctional irrigation device comprising an inner wall for securing a cap valve.
Figure 27 shows a cross-section of a cap valve.
Figure 28 shows a cross-section of the cap valve depicted in Figure 27 secured to the inner wall of the inventive embodiment depicted in Figure 26.
Figure 29 shows a cross-section of a cap valve with two openings.
Figure 30 shows a cross section of a irrigation device having two conduits.
Figure 31 shows a cross section of the cap valve of Figure 29 secured to the irrigation device depicted in Figure 30 in an open orientation.
Figure 32 shows a cross section of the cap valve of Figure 29 secured to the irrigation device depicted in Figure 30 in a closed orientation.
Figure 33 shows a cross-section of a cap valve with two openings and a perforated edge.
Figure 34 shows a cross section of a irrigation device having two conduits and a perforated inner wall.
Figure 35 shows a cross section of the cap valve of Figure 33 secured to the irrigation device depicted in Figure 34 in a closed orientation.
Figure 36 shows a cross section of the cap valve of Figure 33 secured to the irrigation device depicted in Figure 34 in an open orientation.
Figure 37 shows a further inventive embodiments of a multifunctional irrigation device comprising multiple indirect stream nozzle outlets.
Figure 38 shows the device of Figure 37 with a further inventive embodiment of a cap valve.
Figure 39 shows a further inventive embodiment comprising a cap valve in an direct stream configuration.
Figure 40 shows a further inventive embodiment comprising a cap valve in an indirect stream configuration.
Figure 41 shows the irrigation device depicted in Figure 40 with the cap valve removed.
Figure 42 shows the irrigation device depicted in Figure 39, including the shield omitted from Figure 39.
Figure 43 shows the irrigation device depicted in Figure 40, including the shield omitted from Figure 40.
Figure 44 shows the irrigation device depicted in Figure 41, including the shield connector omitted from Figure 41.
Figure 45 shows an inventive embodiment of an irrigation device having a flexible distal shield edge in a straightened, relaxed position.
Figure 46 shows the embodiment of Figure 45 with the flexible distal shield edge in a flexed position.
Figure 47 shows an inventive embodiment of an irrigation device having a flexible distal shield edge in a curved, relaxed position.
Figure 48 shows the embodiment of Figure 47 with the flexible distal shield edge in a flexed position.

### DETAILED DESCRIPTION

Irrigation devices and methods are disclosed herein which incorporate specialized components and features within to allow for multifunctional irrigation.

### IRRIGATION DEVICES

Generally, irrigation devices and systems disclosed herein can comprise a fluid source connector configured to connect to a fluid source, and a shield configured to prevent splashing and contain the irrigation fluid. The fluid source connector can comprise a first nozzle, the nozzle comprising a nozzle inlet within the fluid source connector, and a nozzle outlet outside of the fluid source connector (e.g., within the shield). The first nozzle can thereby form a conduit which extends through the fluid source connector to deliver irrigation fluid from the irrigation fluid source and ultimately to the tissue to be irrigated. In certain aspects, the first nozzle can be positioned within a partition of the connector, the partition separating the fluid source connector from the space external to the fluid source connector.

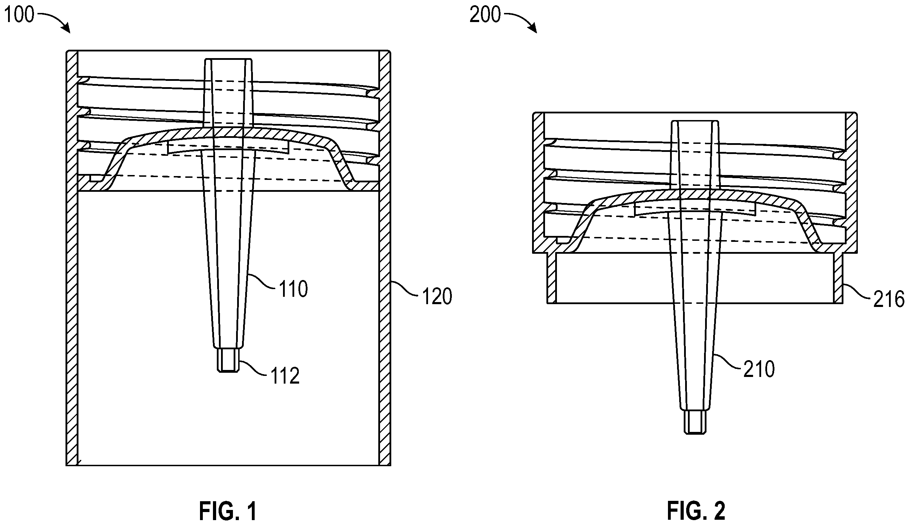

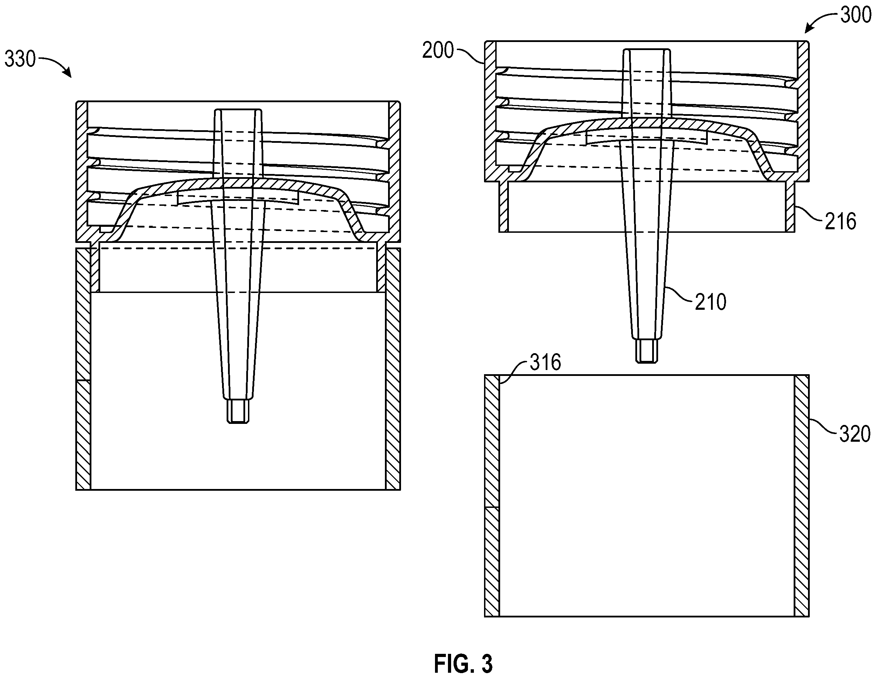

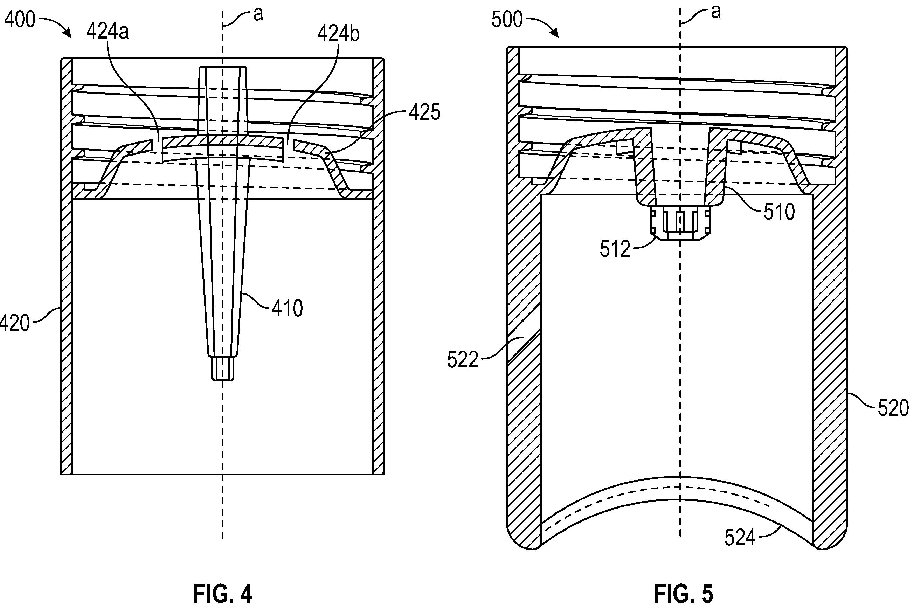

Irrigation devices disclosed herein can operate under multiple irrigation functions, where previously only single function adapters were possible. For instance, irrigation devices disclosed herein can be configured to deliver a stream of irrigation fluid directly and indirectly depending on the configuration of the device. In certain aspects, the irrigation device can comprise a direct stream nozzle outlet configured to deliver irrigation fluid through the device against the shield, and not against the tissue to be irrigated. As will be understood by those of ordinary skill in the art, indirect streams may be particularly useful for sensitive tissues where penetrating flow is not desired, advantageous, or required, such as for ocular irrigation. In these and other aspects, the irrigation device can further comprise a direct stream nozzle outlet configured to deliver a stream of irrigation fluid through the device and contacting the tissue to be irrigated directly. As will be understood by those of ordinary skill in the art, direct irrigation stream may be particularly useful where the irrigation fluid is intended to perturb the tissue, such as in wound, abscess, or ear irrigation. Prior art single function irrigation devices are depicted herein for reference. Figure 1 shows a wound irrigation shield of the prior art that fits on a bottle or on the end of a syringe to retain fluid within the shield when applied to a surface wound of the skin. Figure 2 shows an abscess irrigation shield of the prior art that fits on a bottle or on the end of a syringe to retain fluid within the shield when applied to a deep pocket of a wound below the surface of the skin. Figure 3 shows a wound irrigation shield of the prior art that fits on a bottle or on the end of a syringe to retain fluid within the shield when applied to a surface wound of the skin and with a removable extension shield so that the device can also be used for the irrigation of an abscess below the surface of the skin. Figure 4 shows a wound irrigation shield of the prior art that fits on a bottle or on the end of a syringe to retain fluid within the shield when applied to a surface wound of the skin with a "straight" nozzle. Figure 5 shows an eye irrigation shield of the prior art with a saddle rim that helps to form a seal over the periorbital surface.

In each of these prior art embodiments, the devices are limited by features that result in advantages that contradict or interfere with the operation of the device for alternate irrigation methods. For instance, the embodiments of Figures 1 and 2 are shown as alternatives for the irrigation of wounds and abscesses. As shown, wound irrigation device 100 has a central nozzle 110 that terminates at nozzle outlet 112 within shield 120, so as to prevent splashing of the irrigation fluid after contacting the wound. However, the shielded arrangement of wound irrigation device 100 prevents its use for abscess irrigation, as central nozzle 110 cannot extend beyond the shield 120 and into an abscess cavity. Figure 2 presents an abscess irrigation device 200, where central nozzle 210 is able to extend into an abscess cavity and irrigate the tissue. Abscess irrigation device 200 is shown with shield connector 216, but lacking a shield connected thereto to allow the central nozzle 210 to extend into the abscess.

Figure 3 shows a prior art example of a multifunctional irrigation system 300 for the irrigation of wounds and abscesses. Multifunctional irrigation system 300 comprises abscess irrigation device 200 with removable shield 320, which can be optionally secured to shield connector 216 at inner surface 316 of the shield 320. Once shield 320 is attached, the device as constructed represents wound irrigation device 330. Of course, the shield may again be removed to return the device to an abscess irrigation device 200. When attached to the device, the removable shield may allow optimal configuration for wound irrigation as for Figure 1. When removed from the device, the device results in a configuration that is optimal for abscess irrigation. Irrigation device 400 shown in FIG. 4 is designed to deliver a high pressure wound irrigation stream inline with axis a of the device 400 and directed at the center of the area encompassed by the shield 420 to blast microorganism and contaminants off the wound surface. Partition 425 of irrigation device 400 also contains fluid outlets 424a and 424b each of which also serve as and generate a single straight direct stream aimed at the wound area encircled by the shield 420. The stream exiting nozzle 410, and either of fluid outlets 424a and 424b can be inline with central axis a of the shield when used with a syringe and multiple direct streams aimed at the wound area encircled by the shield and inline with axis *a* of the shield 420 when the shield 420 is threaded onto an irrigation bottle. As will be appreciated by those of skill in the art, the prior art device 400 depicted in FIG. 4 is not optimal for eye irrigation given the risk of injury of the eye surface with the high impact pressure to the delicate eye surface and the triggering of the blinking reflex that would prevent access to the injured eye surface tissue when the eyelids are closed due to the blinking reflex.

Eye irrigation device 500 shown in Figure 5 fits on a bottle to retain fluid within fixed shield 520 when applied to a surface wound of the skin. Device 500 contains a central nozzle terminating at an indirect stream nozzle outlet 512, creating a deflected stream that avoids delivering a high pressure wound irrigation stream inline with the axis *a* of the shield. The indirect stream is purposefully designed not to blast the surface of the eye and avoid injuring the delicate eye surface and to avoid impact that would trigger the blinking reflex. The nozzle outlet 512 has multiple lateral openings that direct fluid to the inner surface of the surrounding shield 520 so that it trickles down the wall of the shield 520 and pools up to create a bath of irrigation fluid over the surface of the eye without any direct impact that could harm the delicate surface of the eye or trigger the blinking reflex that would prevent access to the washing the injured eye surface. The device is not designed for high impact irrigation that is used for wound irrigation in emergency room settings.

In the dedicated eye irrigation embodiment shown in Figure 5, saddle rim 524 helps retain the fluid so that it pools up to bathe the surface of the eye to dilute any irritants or wash away any debris. Overflow outlet 522 through the thick wall that drains overflow fluid to permit continual and interrupted dilution with the application of fluid to the bath. Overflow outlet 522 aims fluid downward to avoid squirting fluid outward and thereby reducing the treatment area and the mess. associated with the procedure. The blunt thick-walled shield 520 is more gentle to the periorbital surface of the injured eye, but the thick wall of the part makes the part more costly to manufacture due to the increased transparent plastic material that is required and the increased cooling time required when the part is injection molded. The monolithic structure reduces the need for assembly and keeps the nozzle in a recess position safely above the eye surface.

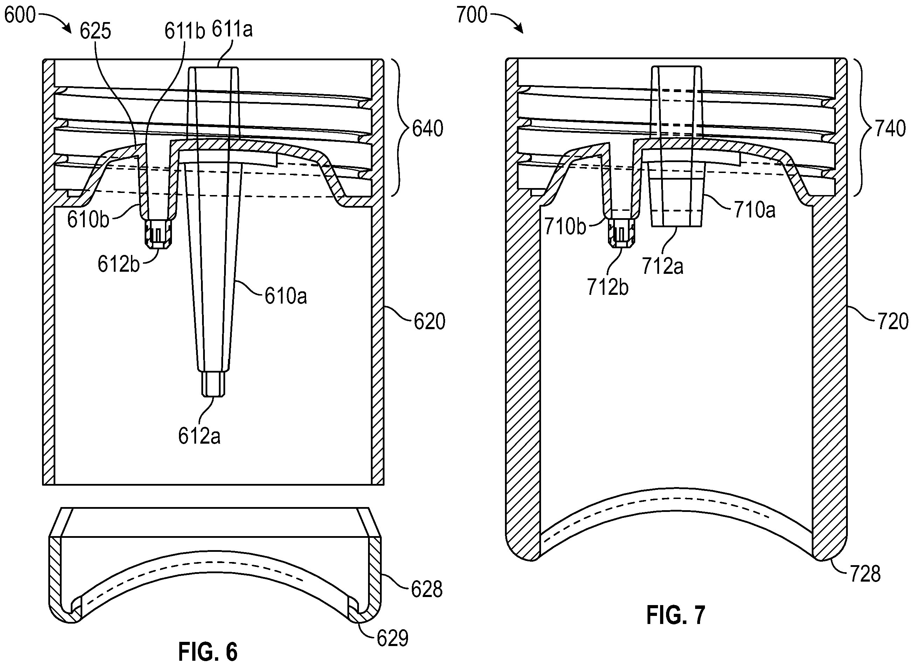

Inventive embodiments are disclosed herein which adapt the features and configurations of single-function irrigation devices into a compatible multifunction device, where combination of such functions has previously been found incompatible or disadvantageous. For instance, Figure 6 shows an inventive embodiment of an irrigation device that can be used for both safe eye irrigation and effective wound irrigation.

As in prior art devices, device 600 comprises a fluid connector 640 separated from a shielded volume by partition 625. Unlike prior art devices, device 600 shown in Figure 6 comprises multiple nozzles extending through the partition. Device 600 has a first nozzle 610a with a direct stream nozzle outlet 612a that creates a direct stream that is substantially aligned with the central axis of the nozzle 610a. Preferably the stream is a single concentrated stream to generate a maximum pressure at a single location. Device 600 also has a second nozzle 610b with a direct stream nozzle outlet 612b that creates an indirect stream that is substantially orthogonal to the central axis of the nozzle 610b.

Device 600 comprises a fixed, thin-walled shield 620. However, device 600 also comprises a removable saddle rim adapter 628 that can be selectively secured to the distal end of shield 620 during eye irrigation procedures. As shown, saddle rim adapter 628 can be formed from a thin-walled member but curved to present a contacting edge 629 presenting a similar shape and thickness to the periorbital surface. Contacting edge 629 helps to form a seal over the periorbital surface. The embodiment is designed not to blast the surface of eye to avoid injuring the delicate eye surface and to avoid impact that would trigger the blinking reflex when using the spray stream nozzle with the indirect stream.

Device 600 can further comprise caps or plugs, not shown, to selectively seal or cover nozzle inlets 611a, 611b, and/or nozzle outlets 612a, 612b. There may be at least one open nozzle and one closed nozzle to provide an indirect stream for eye irrigation, preferably with the attachable saddle rim adapter 628. There may be at least one open nozzle and one closed nozzle to provide a high pressure stream, preferably within the center of the shield, depending on the needs of the user and the patient. For example, a plug in the nozzle 610b only would permit a low impact indirect stream. A cap over the spray nozzle 610a only would permit a high impact stream for optimal surface would irrigation. A user could select whether they want a flat rim as present on shield 620 without saddle rim adapter 628 for better containment of fluid on a flat body surface or a saddle rim by attaching saddle rim adapter 628 to shield 620 for better containment of fluid over the eye.

Figure 7 shows a further embodiment of an irrigation device 700 having two types of irrigation nozzles with a single bottle connector, as in the prior figure. In this embodiment the wide saddle rim 728 is shown as being monolithic with the shield 720 extending from the fluid source connector 740. In this embodiment, indirect stream nozzle 710b extends beyond the end of direct stream nozzle 710a to allow the indirect stream exiting from nozzle outlet 712b to avoid contacting direct stream nozzle 710a. The indirect stream can emit in any direction radial to the central axis of the spray nozzle 710b without such interference. This arrangement avoids the indirect stream dripping down the nozzle 710a over the center of eye and triggering the blinking reflex. Such dripping would counteract the benefits of the indirect eye was stream emanating from the spray nozzle 710b. This embodiment could allow a user to be prepared for optimal eye irrigation and wound irrigation with a single device system. As in the prior embodiment, caps and plugs could be used so that only one type of irrigation mode is used at a time to optimize the procedure for its intended purpose. The product would provide for more common surface wound irrigation but have eye irrigation capabilities for less common but more urgent eye emergencies, thereby providing better emergency preparation and treatment. The device would be stocked and used for more common high impact surface wound irrigation yet have the capabilities for the less common but universally needed eye irrigation. For example, OSHA has requirements for eye irrigation for healthcare work environments due to these universal needs. A wound care center may typically only irrigate surface wounds, but bodily fluids or chemicals might get into a healthcare providers eye requiring immediate rinsing. Having a device that staff is familiar with for wound applications would allow staff to adapt the device quickly for the eye irrigation mode for such uncommon urgent and emergent eye irrigation needs.

The at least two irrigation nozzles shown can have caps or plugs, not shown, to seal or cover all of the inlets and/or outlets, depending on the needs of the user and the patient and to control flow for those needs. There may be at least one open outlet of a nozzle to provide an indirect stream for eye irrigation with a closed obstructed nozzle for higher pressure direct impact. There may be at least one open nozzle preferably in the center of the shield to provide a high-pressure stream, preferably within the center of the shield and one closed spray nozzle to create an indirect stream; when a high pressure direct impact irrigation stream is desired.

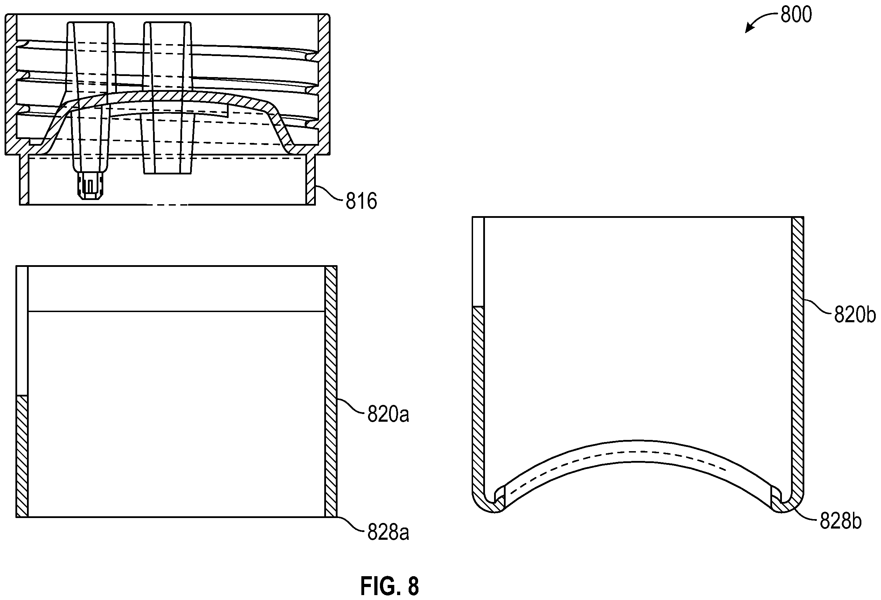

Figure 8 provides a further embodiment of an irrigation system 800 that can be used for safe eye irrigation and also used for wound irrigation. As shown, system 800 comprises the same arrangement of an indirect stream nozzle 810b and direct stream nozzle 810a as in device 700 shown in Figure 7 above. However, system 800 comprises an interchangeably removable extension shields 820a having a flat rim 828a and shield 820b having a saddle rim 828b. Each shield 820 may be connected to shield connector 816, as for further embodiments described above.

Accordingly, irrigation system 800 imparts the ability for a single bottle to generate either a spray stream or a straight direct stream. Device 800 may further have the ability for a fluid source such as a syringe with a luer lock to generate either a spray stream or a straight stream. In this embodiment there are two syringe connectors protruding away from the shield. Prior art embodiments had a single protruding syringe luer connector capable of mating with a syringe a luer lock matched with a single nozzle with a straight outlet that formed a direct stream; or two syringe connects without nozzles which generated only a direct stream. The prior art was not capable of generating indirect stream, such as a stream directed orthogonal toward the inner surface of a shield, with a syringe luer lock connector. The prior art was not capable of using a single wound irrigation shield device with a syringe with a luer lock to irrigate with an irrigation nozzle with straight outlet forming a direct stream and an irrigation nozzle with an indirect stream. This embodiment allows a user to use a common luer lock syringe to form either an indirect spray stream such as an orthogonal stream direct toward the inside of the shield for eye irrigation or a high impact direct stream such as one down the center of the shield opening depending on their needs. Furthermore, using embodiments with the threaded bottle connector and syringe connectors, either syringe and bottles could both be used to generate either a high-pressure direct impact irrigation stream or at least one indirect "no" impact irrigation stream aimed at the inner shield wall. The increased versatility would allow for better preparation and readiness for both routine wound irrigation procedures and emergent eye irrigation procedures with a variety of devices depending on the availability of the equipment, needs of the patient and preference of the healthcare provider. In certain aspects, such as embodiments comprising two independent nozzles, irrigation devices may further comprise caps or plugs, not shown, to selectively seal or cover nozzle inlets and/or nozzle outlets, depending on the needs of the user and the patient and to control flow for those needs.

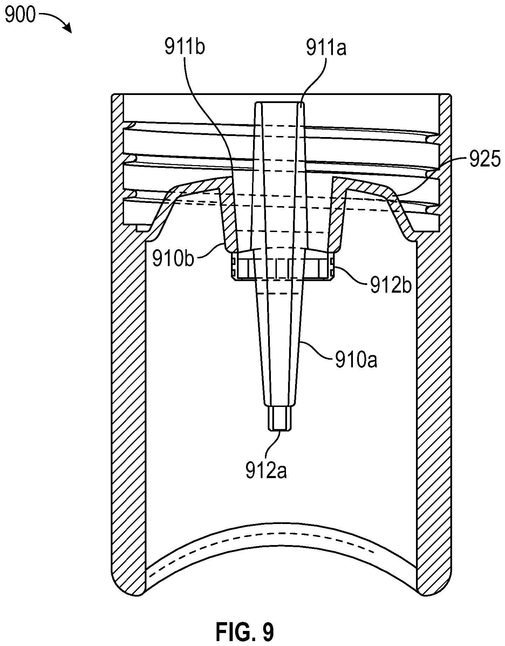

Figure 9 shows an irrigation device that can be used safely for both eye irrigation and wound irrigation. Unlike eye irrigation devices of the prior art, the embodiment of Figure 9 features concentric nozzles 910a, 910b to alternatively generate an indirect stream and a direct stream of irrigation fluid. As shown, device 900 comprises a first nozzle inlet 911a extending as nozzle 910a and terminating at direct stream nozzle outlet 912a. Device 900 further comprises a second nozzle inlet 911b, shown as a recess within partition 925 configured to deliver irrigation fluid through nozzle 910b, and terminating at indirect nozzle outlet 912b. Device 900 thereby creates both an indirect stream and that is substantially orthogonal to the axis of the nozzle 910b and a high-pressure irrigation nozzle with a direct stream outlet 910a for direct high impact irrigation. Unlike irrigation bottle shields with indirect streams used for eye irrigation in the prior art, this device can also be used with a bottle or a syringe for high pressure direct impact irrigation. The spray or indirect stream outlets 912a, 912b are placed concentrically to the axis of the high impact direct stream irrigation nozzle with the straight outlet. This allows the indirect stream outlets to be at any level along the axis of the high impact direct stream irrigation nozzle with the straight outlet shown in the center of the stream, without any concern for an orthogonally directed stream contacting the nozzle 910a and causing fluid to drip into the center of the eye irrigation field and triggering the blinking reflex.

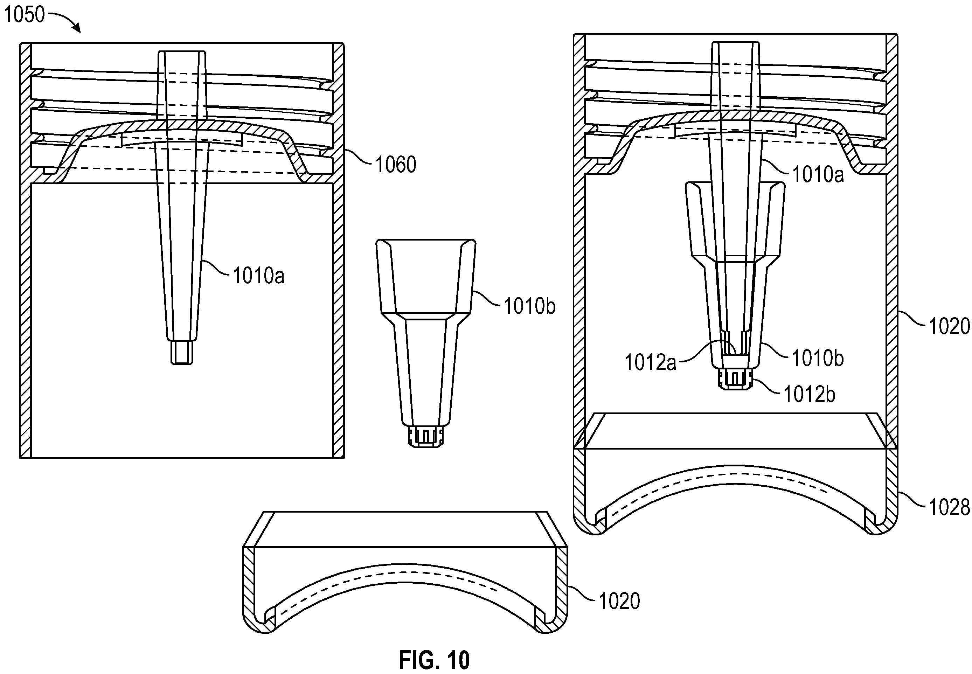

Figure 10 shows a multifunctional irrigation system that can modify a bottle wound irrigation shield of the prior art so that it is improved for eye irrigation. The fully assembled device 1050 comprises a removable saddle rim 1028 to convert may convert a flat rim into a contoured rim that is better able to seal and trap fluid over the periorbital region and within a shield when necessary for eye irrigation. An attachable second nozzle 1010b with at least one indirect stream nozzle outlet(s) 1012b that can generate an indirect stream toward the side of irrigation shield 1020 can redirect a direct stream exiting the first nozzle 1010a at nozzle outlet 1012a for high impact irrigation into a "no impact" irrigation stream or streams directed at the inside of the shield. The attachable spray second nozzle 1010b may be in a position over the tip of the straight first nozzle 1010a. Alternatively, in arrangement 1060, the device is configured for wound irrigation having second nozzle 1010b removed from the first nozzle 1010a.

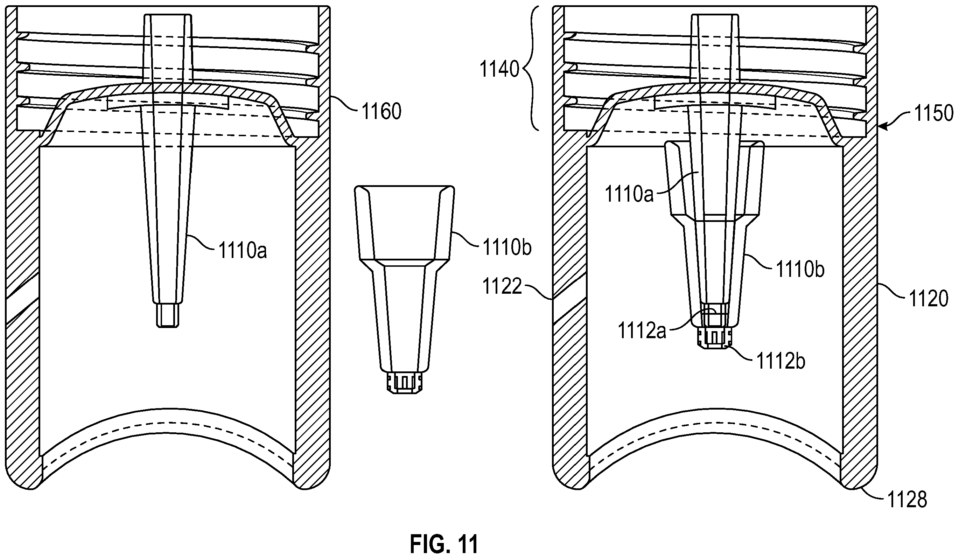

Further embodiments are disclosed which allow both optimal eye irrigation and optimal surface wound irrigation without the expense and bulk of having two threaded parts with shields with nozzles that are primarily designed and dedicated for a mode or function. Figure 11 for instance, shows a multifunctional irrigation system embodiment comprising a fixed saddle rim. In the assembled embodiment 1150, the saddle rim 1128 is monolithically connected to the shield and monolithically connected to the threaded bottle fluid source connector 1140 and monolithically connected to the first nozzle 1110a. An attachable second nozzle 1110b with at least one indirect stream nozzle outlet 1110b that can generate an indirect stream toward the side of irrigation shield 1120 can redirect a straight first nozzle 1110a for high impact irrigation into a "no impact" irrigation stream or streams directed at the inside of shield 1120. The attachable spray second nozzle 1110b may be positioned over the tip of the straight first nozzle 1110a. The system may have as few as two parts and would have fewer parts than a system with a removable saddle rim. The system may have an overflow port 1122 in the wall of the shield to allow irrigation fluid to exit the shield upon filling the shield cavity during irrigation. As for system 1050, the device may be configured into wound irrigation arrangement 1160 by removing the second nozzle 1110b.

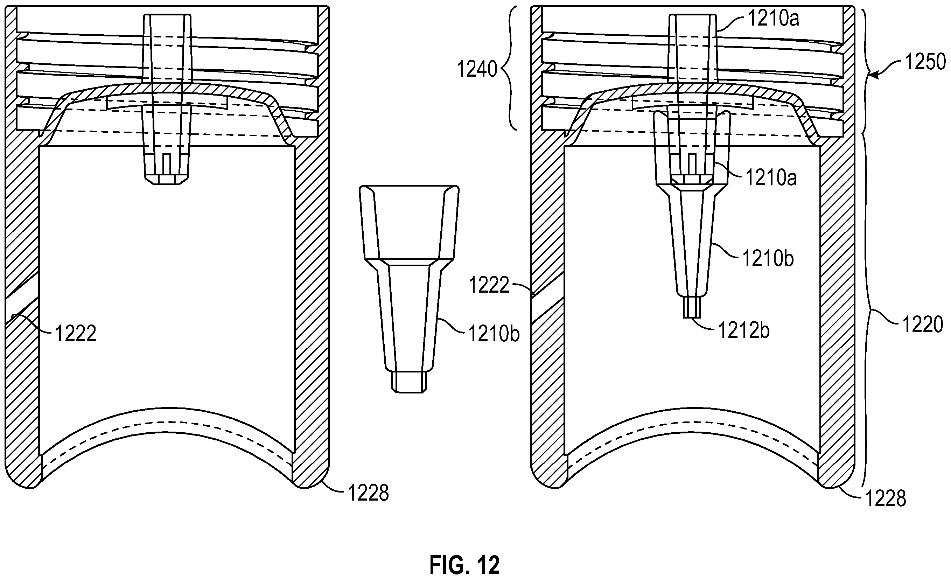

Figure 12 shows a still further embodiment of a multifunctional irrigation system, shown assembled as device 1250 with the first and second nozzles function reversed, and comprising a fixed saddle rim. In this embodiment the saddle rim 1228 is preferably monolithically connected to the shield 1220 and monolithically connected to the threaded bottle connector 1240 and monolithcally connected to the first nozzle 1210a. It will be understood that embodiments comprising a detachable saddle rim are also contemplated. In this embodiment, second nozzle 1210b has a direct stream nozzle outlet 1212b, and can generate a direct stream of irrigation fluid directed toward a wound surface without first contacting a wall of the shield 1220. The attachable second nozzle 1210b can redirect the sprayed fluid of a first nozzle 1210a and change its pattern from a multiple streamed orthogonal stream into a straight single concentrated stream. In certain aspects, the second nozzle 1210b can be preassembled onto the first nozzle 1210a for common high-pressure irrigation of a wound using a bottle or a syringe. When there is an eye emergency requiring eye irrigation, the second nozzle with the straight outlet generating the high-pressure stream toward could be removed to expose the first nozzle 1210a with the indirect stream nozzle outlet 1212a which would be more suitable for use for safe and effective eye irrigation. In this embodiment, the first nozzle outlet 1212a is preferably recessed within the shield that is monolithically attached to the first nozzle to prevent the tip of the nozzle from impacting on an eye surface during irrigation. Having the shield 1220 monolithically attached avoids the risk of this shield from coming loose and losing the nozzle protecting function. In further aspects, the direct stream second nozzle 1210b can be preassembled over the first nozzle 1210a. This advantageously reduces the number of loose parts and allow a user to simultaneously remove all the components of the system 1250 at the same time from a package as a single unit even without the second irrigation nozzle 1210b being monolithically being attached.

Moreover, the removal of the nozzle lengthening tip would shorten the overall length of an exposed nozzle tip to the base of the first nozzle and therefore the overflow outlet 1222 of the eye irrigation shield could be positioned higher in the wall of the shield without the spray tip extending past it. As shown, the positioning of the overflow outlet within the shield distal to the termination of the indirect stream nozzle outlet 1212a allows the nozzle outlet 1210a to perform as intended without becoming submerged within the shield during use. The abbreviated termination of the indirect stream nozzle outlet 1212a also allows a larger volume of fluid to be retained in the shield 1220 before draining which would help with the initial dilution of the fluid and would be of benefit in completely covering the eye with fluid, for instance when a patient's head is rotated to the side of the drain. Accordingly, the disclosed arrangement in Figure 12 provides the unexpected advantage to eye irrigation of increased volume and improved flow of the irrigation fluid through the device during operation. Of course, the direct stream second nozzle 1210b will retain the respective advantage of having a nozzle outlet 1212b extended toward the tissue during wound irrigation.

The system 1250 of Figure 12, and others disclosed herein, can be used in an emergency room setting frequently for wound irrigation, and have the added benefit of being immediately available for conversion to an eye irrigation device with removal of a single part. In contrast, a dedicated eye irrigation product is less likely to be used in an emergency room setting than a wound irrigation shield with a straight nozzle (for example if there are more than 10x the number of lacerations treated in emergency rooms than eye injuries). Therefore, a dedicated eye irrigation device is less likely to be used and staff may be unfamiliar with its use or its stocking location. However, a frequently used product such as the multifunctional embodiment would be more familiar to staff and the clinical staff would be better prepared to start treatment of an eye injury with a reduced risk of delays.

In certain aspects, the irrigation device may comprise one or more flexible nozzles. For instance, the first nozzle and/or second nozzle can be flexible to aid in a more secure pressure fit of the second nozzle over the first nozzle. Additionally, a soft flexible nozzle that is softer than the rigid plastic of a transparent injection molded wound irrigation shield made of a stiff plastic, e.g. polycarbonate, may pose less risk of harming the eye if the nozzle came loose should it have been used improperly during eye irrigation. In certain aspects, the second nozzle may also be of a different transparency, tint or color than the first nozzle to highlight the removability.

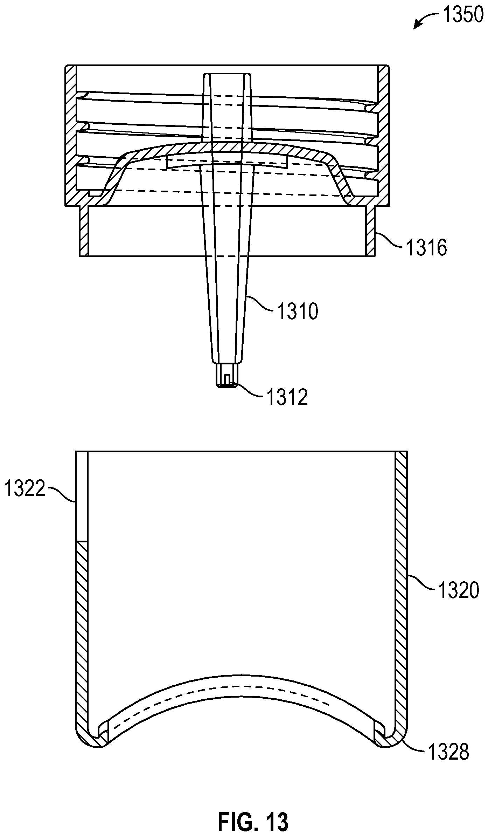

Figure 13 shows multifunctional irrigation system 1350 comprising a first nozzle 1310 with an indirect stream nozzle outlet 1312. The system 1350 can be coupled with a removable shield 1320 and a saddle rim 1328 suitable for eye irrigation. As shown without the shield 1320 attached, the nozzle 1310 extends beyond a shield connector 1316 and therefore can penetrate into an abscess for abscess irrigation by flooding the abscess to dilute out microorganism, pus and debris. A high-pressured straight stream has less benefit over an indirect stream in a fluid filled abscess pocket than when used on the outer surface of the skin, e.g. when irrigating a laceration compared to a spray stream such as a spray stream from a spray nozzle that generates at least one stream oriented orthogonally from the axis of the nozzle. Likewise, irrigation in the abscess is essentially a blind procedure, since you cannot see deep within the abscess vs irrigating a surface wound such as a laceration of the outer skin. Therefore, the targeting benefits of a single concentrated stream from a "straight" outlet of a high-pressure nozzle have a reduced benefit.

As such, a device 1350 with an irrigation device with a shielded spray nozzle 1310 that would be inappropriate for a surface wound irrigation, could be used for abscess irrigation. Such an abscess irrigation device could be combined with a removable shield 1320 and a saddle rim 1328 adapted for improved eye irrigation. While irrigation devices with a protruding nozzle that extends beyond a shield with spray nozzle would not normally be used for eye irrigation due to the risk of the nozzle tip injuring the eye, when combined with a shield 1320 surrounding the nozzle tip, the shield would cause the spray tip to be recessed with reduced risk of harm, if recessed enough and if the shield is stiff enough to prevent compression.

It is also contemplated herein that the removable shield 1320 could form a fluid outlet pathway 1322 for the overflow of fluid and continual dilution caused by the bath created by the sealed of the contoured rim. An embodiment provides a device capable of eye irrigation with a bottle; with a syringe; and with a syringe or a bottle, thereby increasing the options for applying fluid with a fluid source to an injured eye surface in an emergency. Also having a multifunction eye irrigation device would make it more familiar and more readily available to staff that uses it for eye irrigation and other procedures like abscess irrigation.

Embodiments may have a removable second irrigation nozzle that may be pre-attached to the device and may be pre-assembled over first irrigation nozzle that may not be removable. Embodiments may have the spray nozzle preassembled so that it is available for use in emergent situations for self-use when vision is limited and when self-assembly would be difficult. Even when not used for self-use, the pre-assembly of the device for emergent use would speed and simply treatment, such as when there is a splash injury to the eye.

Advantages of the current embodiments include having the capability of using either bottle or a syringe for either high flow/high pressure irrigation with a direct high impact stream; or an indirect stream more suitable for eye irrigation. Either or both of the two components could retrofit with an existing device to provide new capabilities to improve preparation and treatment of eye emergencies. The use of the second nozzle over the first nozzle arrangement allows one central syringe connector to be used. This is much easier to mold for a plastic part with internal threads than having two independent non-concentric nozzles inlets. The embodiments shown also overcome the problems that would be seen if a first elongated nozzle blocked a stream from a second nozzle with an indirect stream such as an orthogonal stream aimed at the inside of a shield wall for eye irrigation.

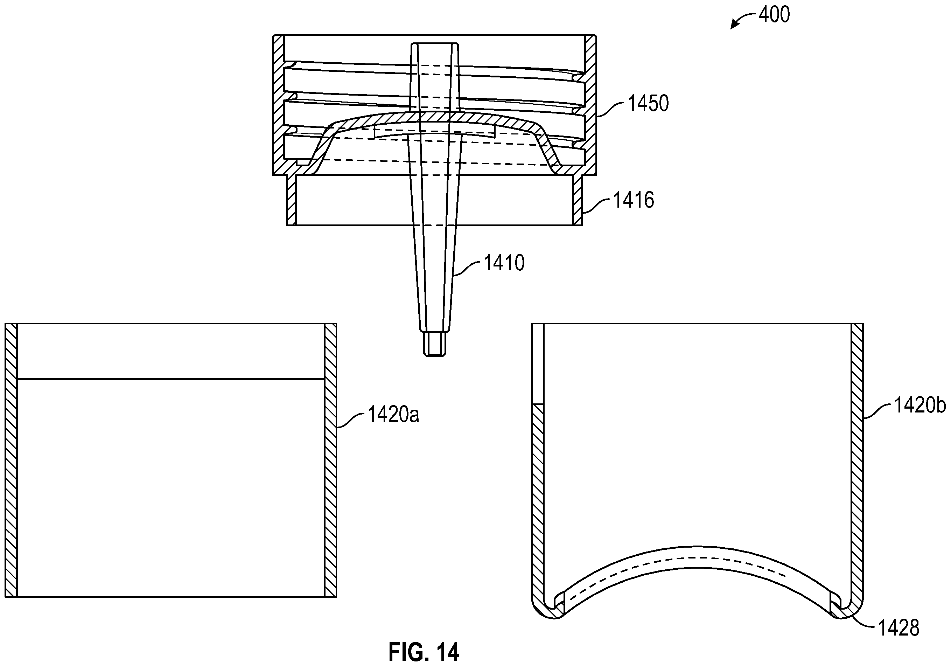

Figure 14 shows an embodiment of a multifunctional irrigation system 1400 comprising a multiple and interchangeable removable shields 1420a and 1420b. The fluid source connector 1440 and nozzle 1410 extending therefrom beyond a shield connector 1416 and can penetrate into an abscess for abscess irrigation by flooding the abscess to dilute out microorganism, pus and debris. When shield 1420a is attached at the shield connector 1416, the device 1450 is converted to a wound irrigation device. Alternatively attaching shield 1420b configures the device 1450 into an eye irrigation device. As for other embodiments disclosed herein, the contoured saddle rim 1428 makes the device easier to create a seal over the eye to create a pool for irrigation of the eye.

As such, a device with a nozzle extending beyond a shield that would typically be inappropriate could be used for eye irrigation. Such an abscess irrigation device could be combined with a removable shield and a removable saddle rim adapted for improved eye irrigation. While a device with a protruding nozzle that extends beyond a shield with spray nozzle would not normally be used for eye irrigation due to the risk of the nozzle tip injuring the eye, when combined with a shield, the shield would cause the nozzle tip to be recessed with reduced risk of harm, if recessed enough and if the shield is stiff enough to prevent compression.

The removable shield could form a fluid outlet pathway for the overflow of fluid and continual dilution caused by the bath created by the sealed of the contoured rim. An embodiment provides a device capable of eye irrigation with a bottle; with a syringe; and with a syringe or a bottle, thereby increasing the options for applying fluid with a fluid source to an injured eye surface in an emergency. Also having a multifunction eye irrigation device would make it more familiar and more readily available to staff that uses it for eye irrigation and other procedures like abscess irrigation.

Certain embodiments may have at least one removable saddle rimmed shield that extends past at least one nozzle tip. It may have multiple removable shields including at least one removable shield with a saddle rim that extends past the tip of the nozzle. The removable shield may an overflow outlet or form an overflow outlet that retains a pool of fluid and allows drainage of overflowing fluid for continuous irrigation when in use in an attached configuration.

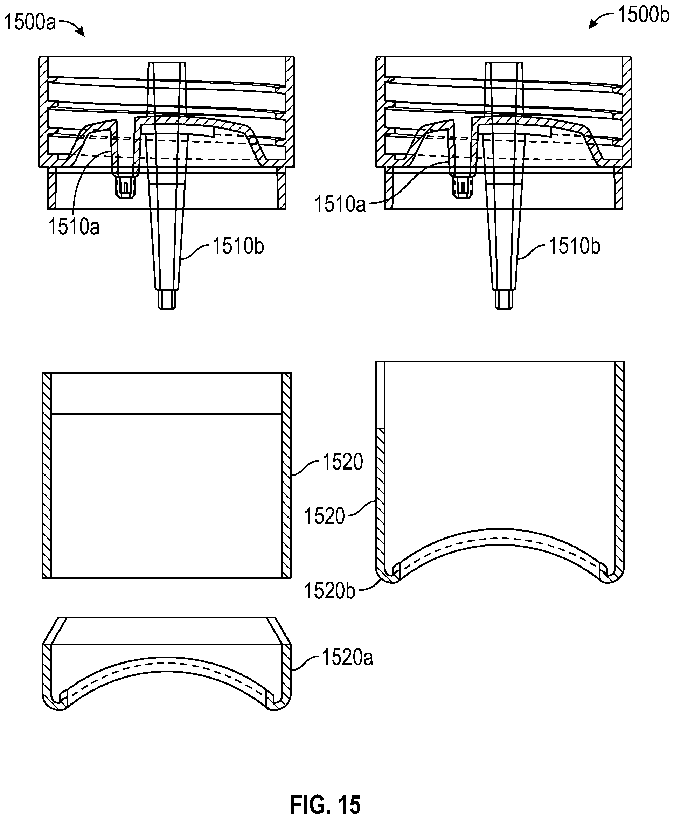

Figure 15 shows alternate embodiments of the multifunctional irrigation systems 1550a and 1550b, each having two types of nozzles, a first nozzle 1510a for an indirect spray stream and a second nozzle 1510b for a high impact direct stream for surface wound irrigation, e.g. for a laceration of the outer skin surface. As above, caps or plugs can be used to control the flow through the nozzles for the desired flow pattern. The saddle rim helps create a seal for creating a pool for eye irrigation. The removable shield 1520 when removed exposes a nozzle extending beyond a shield that can penetrate into an abscess for abscess irrigation by flooding the abscess to dilute out microorganisms, pus and debris. The saddle rim can either be a removable saddle rim 1528a or monolithic saddle rim 1528b, with respect to the shield 1520. In either case, the contoured saddle rim makes the device easier to create a seal over the eye to create a pool for irrigation of the eye. The removable shield can comprise an overflow outlet or form an overflow outlet that retains a pool of fluid and allows drainage of overflowing fluid for continuous irrigation when in use in an attached configuration.

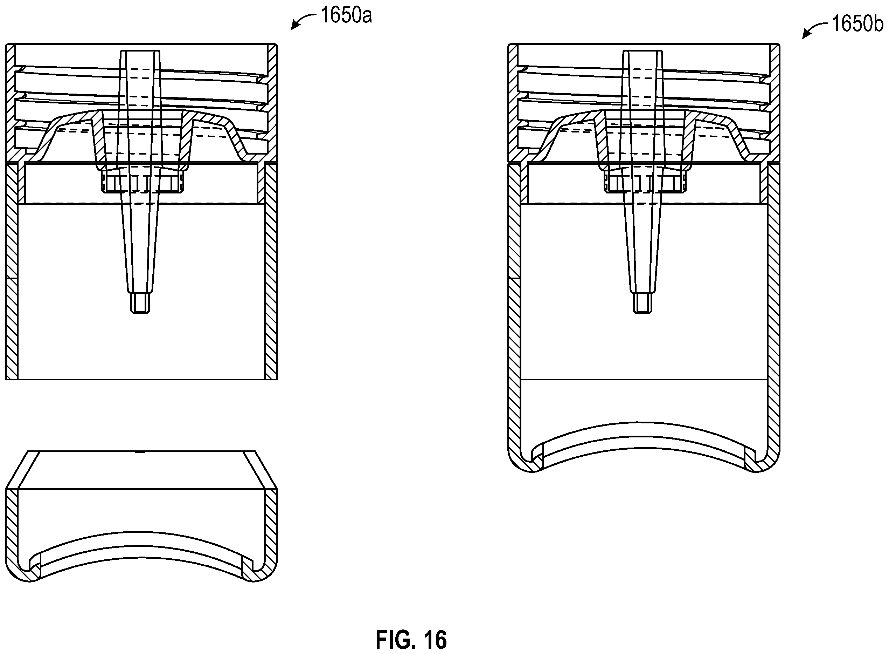

Further embodiments are disclosed as additional combinations and arrangements of advantageous features, each providing unexpected advantages in their implementation as a multifunctional irrigation device. Similar to systems 1550 depicted in Figure 15, the system 1650 of Figure 16 shows embodiments of the invention adaptable to have a fixed or removable saddle rim. Device 1650a can be configured for irrigation of any of a wound, abscess, eye, or even ear irrigation.

Unlike irrigation bottle shields with indirect streams used for eye irrigation in the prior art, embodiments disclosed herein can also be used with a bottle or a syringe for high pressure direct impact irrigation. As shown in Figure 16, indirect stream outlets can be placed concentrically to axis of the high impact direct stream irrigation nozzle. This allows the indirect stream outlets to be at any level along the axis of the high impact direct stream irrigation nozzle with the straight outlet shown in the center of the stream, without any concern for an orthogonally directed stream hitting the nozzle and causing fluid to drip into the center of the eye irrigation field and triggering the blinking reflex. Unlike abscess irrigation devices of the prior art, the embodiments can be used for optimized safe and effective eye irrigation. As for other embodiments herein, a detachable saddle rim may be employed for the embodiment of Figure 16.

The embodiment on the left permits the use of a flat shield for containing fluid effectively on a flat body surface. The embodiment on the right permits the use of a contoured shield for use containing fluid more effectively over a contoured body surface such as the periorbital region of the eye with the safety and convenience of a saddle rim integrated into the removable irrigation shield.

In further embodiments, it is contemplated that the direct stream nozzle may be opened or closed by rotation of the nozzle e.g., as a rotation valve. In such aspects, the rotation of the direct stream nozzle to a closed position may advance the nozzle along the axis of the nozzle, such that indirect nozzle outlets are exposed on a proximal portion of the nozzle. In this arrangement, a single nozzle may be employed to achieve direct or indirect streams without detachment of the nozzle. Alternatively, the nozzle comprising the direct stream nozzle outlet and the indirect stream nozzle outlet also may be detachable from a fixed nozzle, such that detaching the detachable nozzle creates a third nozzle outlet terminating the fixed nozzle.

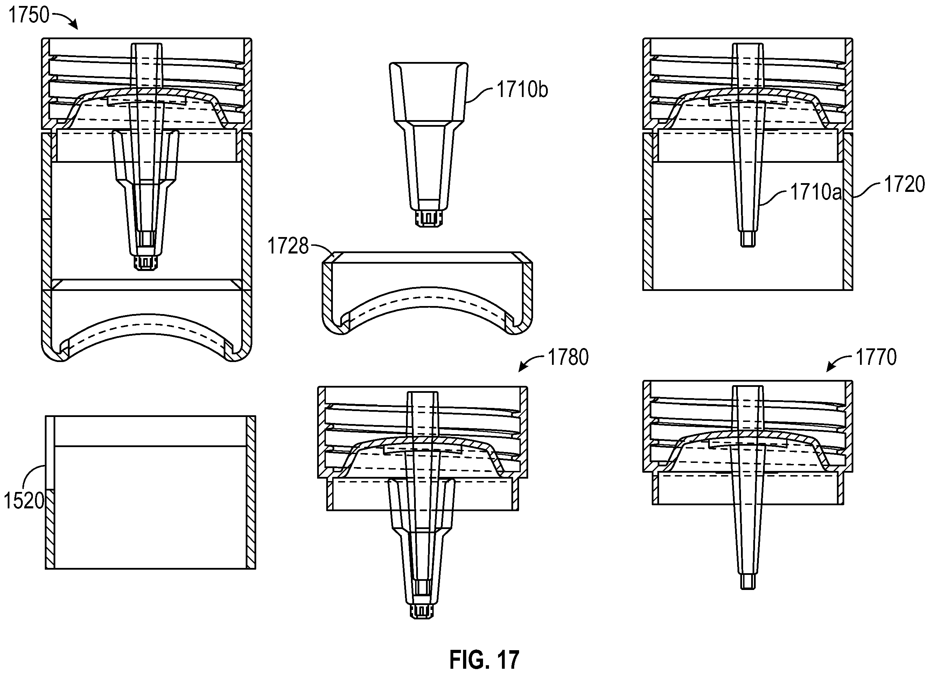

Figure 17 shows a still further embodiment of a multifunctional irrigation system comprising an irrigation device having two types of nozzles, a first nozzle 1710a for an indirect spray stream and a second nozzle 1710b for a high impact direct stream for surface wound irrigation, e.g. for a laceration of the outer skin surface. Assembled in form of 1750, the device can operate as an eye irrigation device. Alternatively, the saddle rim 1728 and second nozzle 1710b can be removed to create a wound irrigation device 1760. The shield 1720 may be removed to then adopt an abscess irrigation device 1770. Removing the shield entirely from the eye irrigation device 1750 provides a second form of an abscess irrigation device 1780 having an indirect stream exiting the nozzle 1710b. Caps or plugs are not necessary to control the flow through the nozzles for the desired flow pattern. As for other embodiments, the detachable saddle rim helps create a seal for creating a pool for eye irrigation. In addition, the entire shield is removable from the irrigation device. When removed, the nozzle is exposed and extends beyond the shield connector. The exposed nozzle can penetrate into an abscess for abscess irrigation by flooding the abscess to dilute out microorganism, pus and debris.

In the embodiment shown in Figure 17, the second nozzle fits over the tip of the first nozzle and deliberately deflects the straight stream into an indirect stream that is aimed at the removable shield when attached. Unlike abscess irrigation devices of the prior art, the device can be used for safe and effective eye irrigation, i.e. with the removable shield attached, wound irrigation with the second nozzle removed, and abscess irrigation with the removable shield removed and either with or without the second nozzle comprising the indirect stream nozzle outlet attached to the first nozzle comprising the direct stream nozzle outlet.

The embodiment uses a small number of assembled components to achieve these multiple functions with as little as three parts. The device is provided in the most commonly used irrigation configuration for emergency room use with a nozzle with a straight outlet to generate high pressure wound irrigation. In an emergency requiring eye irrigation, a single part may be removed, i.e., the second nozzle over the spray nozzle, to convert to a configuration for optimal eye irrigation. When fully assembled, abscess irrigation may optimally be performed by removing the removable shield with the second nozzle attached. When preassembled in this configuration, the eye irrigation mode is ready to used emergently without any modification. The device can be easily modified to a high-pressure surface wound irrigation mode by removing the second nozzle from the first nozzle. Use of these embodiments may have the advantage of not requiring that a user selectively close and open multiple fluid pathways to select and prepare the device for the desired stream pattern. This would reduce the risk of touch contamination especially if the device is used for a sterile procedure. This would reduce the manipulation of the device, which is especially advantageous in an emergency situation, such as splash injuries to the eye, where rapid action is necessary. Moreover, as described above, the first and/or second nozzle may be flexible. With the removal of the shield, the unshielded flexible nozzle may be inserted into a sensitive and delicate ear canal to irrigate the ear canal, e.g. with a syringe attached to the connector. A shield surrounding the nozzle base may help contain the backsplash of fluid.

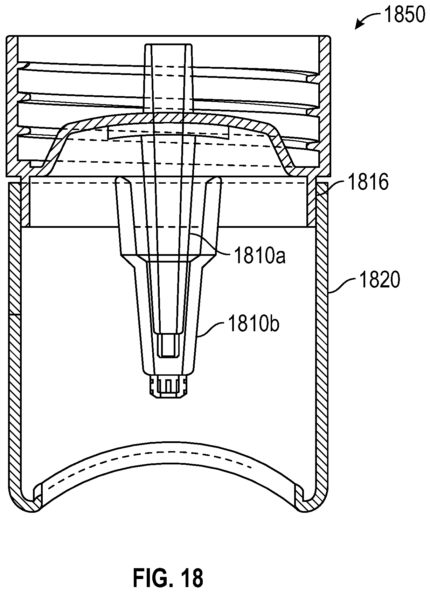

Figure 18 shows a further embodiment 1850 of the invention described in Figure 17, having a fixed saddle rim 1828, and therefore fewer assembled parts while retaining the operation and advantages described above. Particularly, the removable shield 1820 when removed exposes either of nozzles 1810a and 1810b extending beyond the shield connector 1816 that can penetrate into an abscess for abscess irrigation by flooding the abscess to dilute out microorganism, pus and debris. The contoured saddle rim makes the device easier to create a seal over the eye to create a pool for irrigation of the eye. As above, the removable shield may have a pool overflow outlet or form a raised overflow outlet that retains a pool of fluid and allows drainage of overflowing fluid for continuous irrigation when in use in an attached configuration. Unlike abscess irrigation devices of the prior art, the device unexpectedly also can be used for optimized safe and effective eye irrigation, i.e., with the removable shield attached.

The embodiment uses a small number of assembled components to achieve these multiple functions with as little as three parts. An embodiment may be provided in the eye irrigation mode. In an emergency requiring eye irrigation, no modification of the embodiment is needed. When fully assembled, abscess irrigation may be performed optimally by removing the removable shield. The device can be easily modified to a high impact surface wound irrigation mode by removing the second nozzle from the first nozzle. There is no need to selectively plug, unplug, cap or uncap multiple pathways.

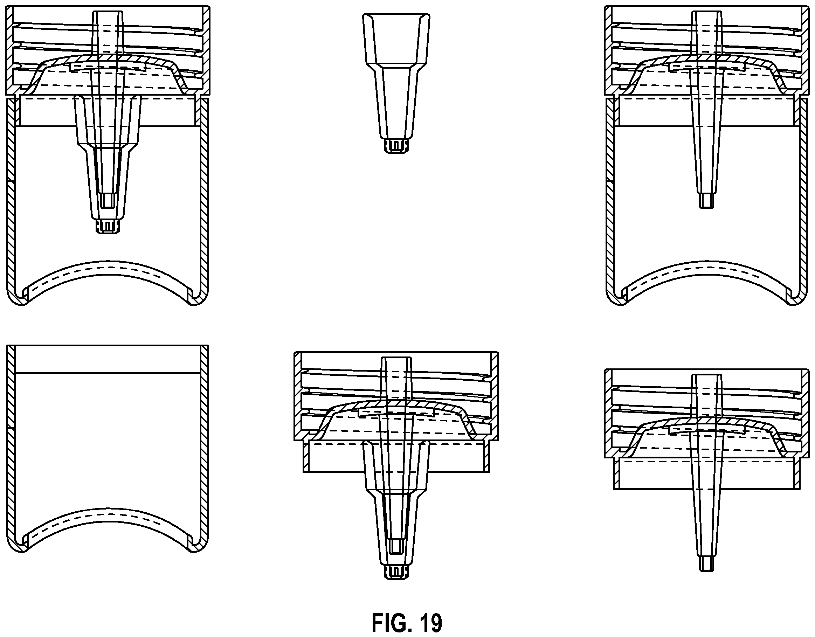

In all aspects described herein, any or all of the nozzles present within the device may be flexible. A soft flexible nozzle, i.e., that is softer than the rigid plastic of a transparent injection molded wound irrigation shield made of a stiff plastic like polycarbonate, would pose less of a risk of harming the eye if the soft flexible nozzle came loose if used improperly during eye irrigation. The second nozzle may also be of a different transparency, tint or color than the first nozzle to highlight the removability of the nozzle. The nozzle may be flexible and with the removal of the shield the unshielded flexible tip may be inserted deep into an ear canal to irrigate a sensitive and delicate ear canal, e.g. with a syringe attached to the connector. Having a nozzle tip that is not surrounded by a shield wall would allow the nozzle tip to be inserted into the ear canal. Having a shield behind the nozzle tip, outside the ear canal, provides the advantage of helping to the block backsplashed fluid while the ear is being irrigated. The nozzle may be flexible and with the removal of the shield the unshield flexible tip may be used to irrigate a sensitive and delicate ear canal, e.g., with a syringe attached to the connector, while having a shield behind the nozzle tip to contain the backsplash of fluid. Figure 19 shows various arrangements of the multifunctional irrigation system described above and shown in Figure 18, analogous to the removable saddle rim embodiments shown in Figure 17.

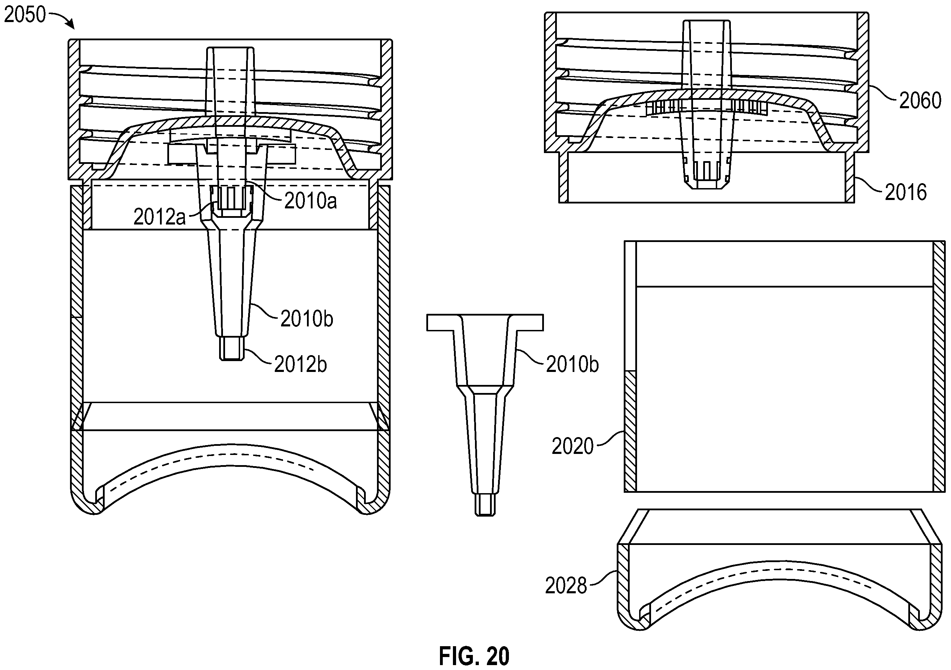

Figure 20 shows a still further embodiment of a multifunctional irrigation system 2050 comprising a first nozzle 2010a comprising an indirect spray stream nozzle 2010a and a second nozzle 2010b removably attached to first nozzle 2010a, the second nozzle 2010b comprising a direct stream nozzle outlet 2012b for a high impact wound irrigation, e.g., for irrigation of a laceration of the outer skin surface. The second nozzle 2010b deliberately deflects the spray stream and focuses it down toward the center of the shield to create a high-pressure central stream. Unlike abscess irrigation devices of the prior art, the device can be used for safe and effective eye irrigation. Unlike optimized eye irrigation devices of the prior art the device can be used for optimized surface wound irrigation with a shield central high-pressure stream through a transparent shield that permits better targeting of the high pressure stream. As best shown in component 2060, the first nozzle may be recessed within the shield connector 2016.

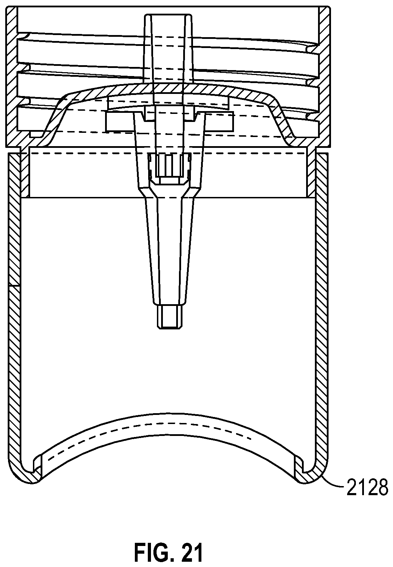

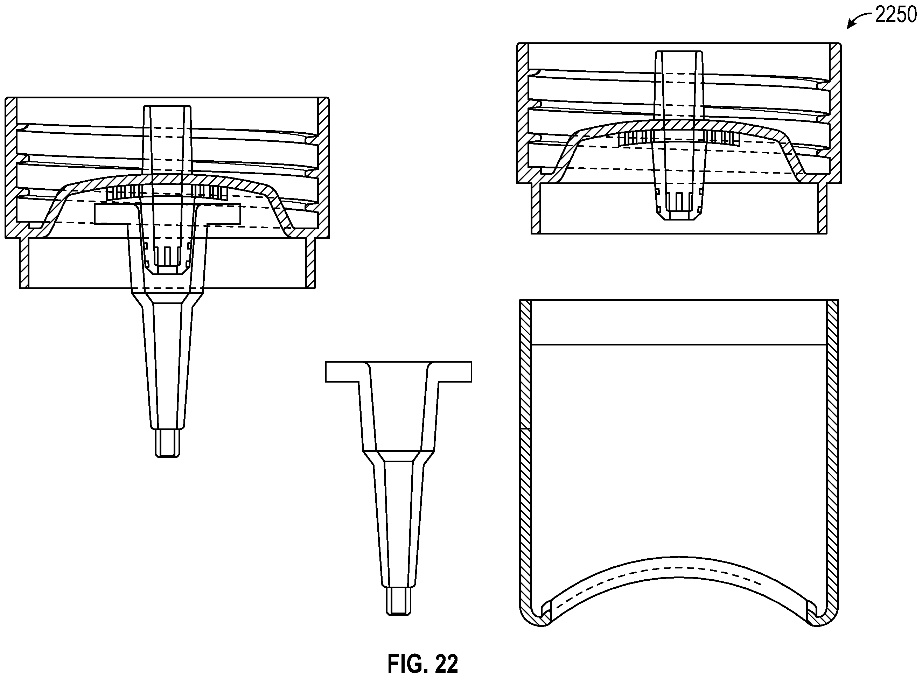

Figure 21 shows another embodiment of the invention comprising two types of nozzles shown in Figure 20, having a fixed saddle rim 2128 as a component of the removable shield. The contoured saddle rim makes the device easier to create a seal over the eye to create a pool for irrigation of the eye. The removable shield can comprise an overflow outlet or form an overflow outlet that retains a pool of fluid and allows drainage of overflowing fluid for continuous irrigation when in use in an attached configuration. Unlike eye irrigation devices of the prior art, device has a nozzle with at least one outlet that creates an indirect stream that is substantially orthogonal to the axis of the nozzle and a high-pressure irrigation nozzle with a straight outlet for direct high impact irrigation. Figure 22 shows the individual components of a system 2250 shown assembled as Figure 21.

As shown in Figure 22, embodiment of multifunctional irrigation system 2250 disclosed herein can use a small number of assembled components to achieve these multiple functions (e.g., as little as three parts). The device of Figures 22 is provided in the most commonly used irrigation configuration for emergency room use with a nozzle with a straight outlet to generate high pressure wound irrigation. In an emergency requiring eye irrigation, a single part may be removed, i.e. the second nozzle over the spray nozzle convert to a configuration for optimal eye irrigation. When fully assembled, abscess irrigation may be performed optimally by removing the removable shield. When preassembled in this configuration, the surface wound irrigation mode is ready to use immediately without any modification. The device can be easily modified to an abscess irrigation mode by removing the shield. Thus, there is no need to selectively plug, unplug, cap or uncap multiple fluid pathways in the device to adopt a particular irrigation function for the device.

When the described embodiment is preassembled in the surface wound irrigation mode it would be used in an emergency room setting frequently for common surface wound irrigation, since wound care accounts for approximately 10% of all ER visits in the US. This configuration would provide the added benefit of being immediately available for conversion to an eye irrigation device with removal of a single part. A dedicated eye irrigation product is likely to used less often in an emergency room setting than a would irrigation shield with a straight nozzle (for example if there are more than ten times the number of lacerations treated in emergency rooms than eye injuries).

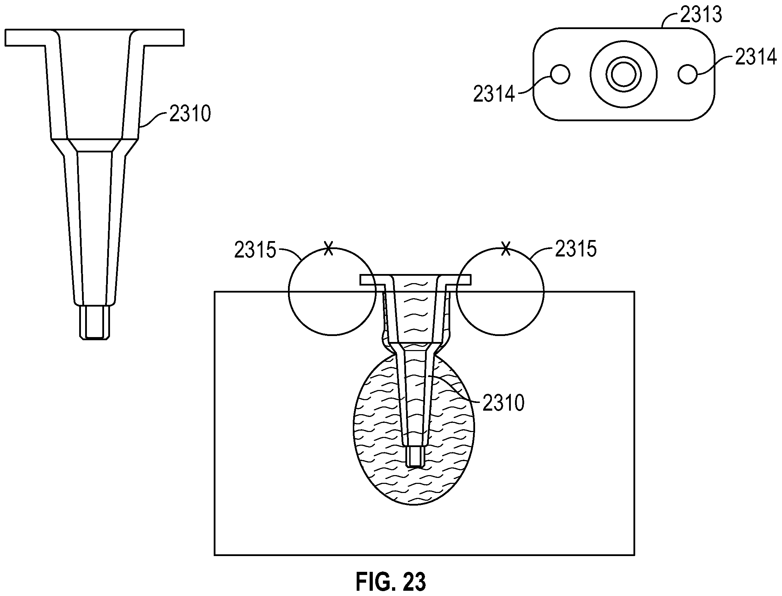

Figure 23 illustrates a removable irrigation nozzle that may also be used as an abscess drain. The nozzle may have features such as ridges, wings or arms, such as extensions 2313 at or near the wider portion, that can help stabilize the nozzle when placed into an abscess. There may be at least one hole 2314 to help restrain the nozzle with at least one suture through the hole to retain the nozzle in place or multiple holes for multiple sutures. The removable nozzle 2310 may be incorporated within any of the previously described embodiments, where appropriate, and could have the benefit of providing a preformed abscess drain with the abscess irrigation device, which could be used after the irrigation process is completed to allow fluid and pus to drain from the opened cavity over a longer period of time and prevent reformation of an abscess at the same site. While abscess irrigation devices do have nozzles, the prior art does not disclose a removable nozzle with retention features used as a preformed drain that is removed from an irrigation device and then intentionally retained, e.g. with sutures, to prevent the dislodgement of the nozzle into a wound accidentally.

A seal opener 2462 may also be incorporated into a fluid source connector 2440, and for any embodiments disclosed herein. Bottles of sterile fluid for wound irrigation are sometimes provided with a seal positioned over the mouth of the bottle. The seal prevents leakage and maintains the sterile integrity of the fluid contents in the bottle. The seal is sometimes provided as a cap liner and applied to the bottle mouth. The seals may be constructed of foam and foil. They may have a tab. They may be constructed from multiple layers in the fashion of the Lift 'n' Peel^{™} brand with a semicircular tab that when unfolded provides a substantially uniform layering height for consistency in applying forces to the rim such as when done with induction sealing. Under emergency conditions, removing the seals can be problematic and interfere with the efficiency and sterility of the irrigation procedure. Seals can be typically the same size as the bottle mouth outer diameter and even if there are tabs on the lids, they are small and difficult to grasp. One style of tab has been constructed from multiple layers in the fashion of the Lift 'n' Peel^{™} brand with a semicircular unfolded tab that when unfolded provides a substantially uniform layering height for consistency in applying forces to the rim such as when done with induction sealing. The seals prevent access to the fluid for wound irrigation.

These disadvantages are exacerbated by the fact that in a clinical environment such as wound care in an emergency room a healthcare provider may have wet gloves which can reduce the ability to grasp and maintain a grip on a small object. Presumably, bottles of wound irrigation fluid, e.g. with 100m to 3000 ml of fluid that have a critical need to have excellent packaging integrity will have a stronger adhesion to a bottle mouth and a thicker more durable more puncture resistant seal than a bottle with less critical contents; and these seals would be harder to remove and puncture, than for example, a tamper evident seal membrane over a bottle mouth of a lightweight bottle containing a lightweight content of candy.

To overcome these disadvantages, sometimes providers may pierce the seal with objects such as forceps, hemostat, scalpel, keys or pens that may be sterile or not sterile. Attempting to pierce the lid may lid to spillage or contamination of the fluid within a bottle. It also adds a step in the preparation for a wound irrigation procedure that complicates preparation, consumes time, and can delay a procedure. It can also lead to an additional sharps risk in a healthcare setting when using a sharp instrument such as scalpel on a tear resistant foam or plastic material. Even when pierced, the liner must still be removed to allowed permit a predictable and uniform circumferential seal that would be expected without the liner, since the disrupted liner will have non-uniform cuts and layers that may interfere with a procedure including impeding fluid flow, fluid pouring, sealing with a cap, blocking flow to an nozzle outlet, or leading to contamination of the fluid in the bottle such as a sterile fluid.

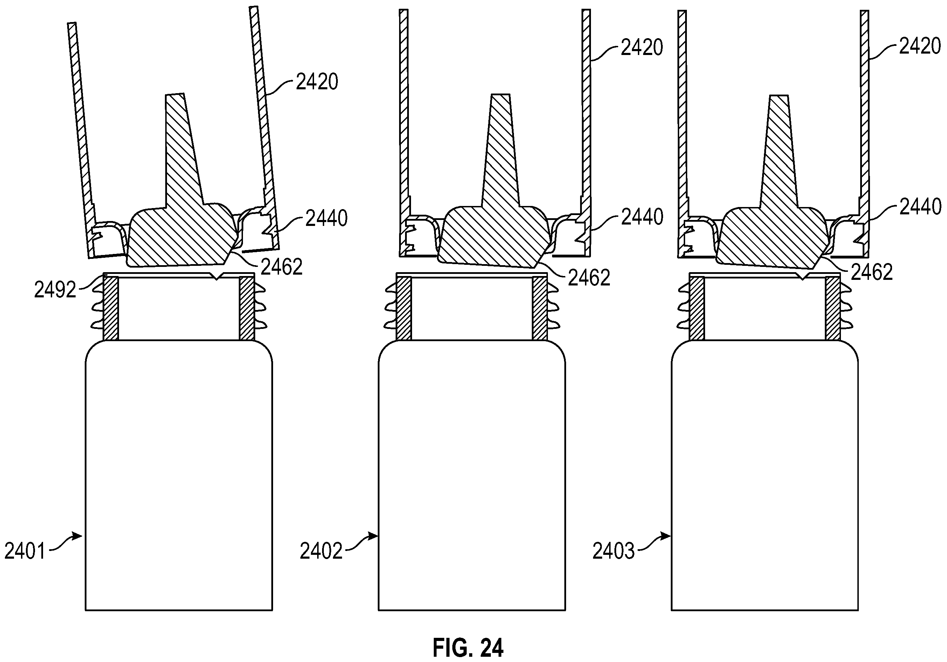

Figure 24 illustrates an embodiment of the present invention with a cap with a blade to pierce the seal of a sterile irrigation bottle with a foam and foil seal, and its attachment to a sealed fluid source in an alignment stage 2401, a pressure stage 2402, and a punctured stage 2403. Throughout the process, the irrigation device may be gripped by the shield 2420, thereby becoming a blade handle by increasing the gripping surface to control the blade for improved gripping during the application of downward pressure and twisting of the blade through the seal. The shield may also protect a nozzle within the shield from contamination during the connection of the device to the fluid source through the seal.

As the shield is advanced onto the threads of the bottle, the blade advances through the seal. In certain aspects, the seal opener may be incorporated into the device as a cap with a threaded inner surface, a nozzle with a seal cutter, a nozzle with a curved blade, a helical blade, an eccentric pointed tip, a beveled tip, a helical ridge form an eccentric point, a helical ridge forming an curved blade, a blade edge widens away from the cutting edge, a non-circular curved blade, a puncturing edge that has a rim that is less than 360 degrees around a nozzle axis. The blade may extend from a ridge that forms a bottle seal. The blade may be located closer to the bottle mouth rim than the bottle mouth center to have a larger opening for pouring, seal removal, or sealing efficiency or for insertion of an apparatus such as a tube with a fluid conduit. The blade may cut less than a complete circular in the seal. The blade may cut a tab in the seal for easier lifting of the seal for removal. The blade tip may be blunt for safety reasons or sharp to improve ease of cutting. The blade may be constructed to cut through a thick layer of material to maintain packaging and sterility integrity that is less frangible than a thin veil of sealing material. The use of the blade with a cap with a nozzle allows the blade to create a hole in the seal as the apparatus, such as a cap with a nozzle, is assembled to a bottle. The hole could be created and flow could occur without having to remove the cap and without having to perform a separate maneuver to assemble the nozzle.

As shown in Figure 24, where the blade extends beyond the edge of the fluid source connector, the seal of the cap 2492 may be punctured before the cap is set over the threads. This configuration may work well with a thin seal that is easily pierced, ruptured or torn since it can be pierced with little force from a variety of positions and a thin easily pierced seal is less likely to interfere with the fitting of a cap over bottle and the creation of a seal. The cap becomes the blade handle. Also, this configuration would be well-suited for use when only a portion of the seal needed to be broken, and the location of the hole is not critical. For example, if only a few drops of fluid are desired and maintaining the sterility is not important, then the configuration and location of the opening in the seal is of less importance. The seal could be punctured and then removed, and the cap could then be placed on the threaded bottle. The seal could be punctured and then the cap could be pressed downward to tear the seal as the cap is threaded in place.

However, the configuration shown in Figure 24 may allow the cap with the blade to be prone to tilting so that the blade may be non-uniform angle or position for which the blade was designed. The blade may be at a poor optimal angle for cutting as pressure is applied with the cap tilted. The blade may slide laterally as pressure is applied with one hand and a bottle is held with another hand. The blade may be positioned over the bottle rim where cutting of the seal will be ineffective. The blade might not extend beyond the threads of the cap so that the blade is less likely to puncture a sterile package when attached to a sterile wound irrigation cap and less like to inadvertently cut someone handling it and less likely to get contaminated when placed on a flat surface.

In addition, when using an embodiment of the invention on a bottle of sterile irrigation fluid with a foam and foil liner, a shielded irrigation cap with a blade that did not extend beyond the threads of the cap was unexpectedly easier to use than a shielded irrigation cap with a blade that extended beyond the threads of the cap. Using embodiments of the invention with an eccentric blade that extended beyond the edge of the cap, the cap blade is prone to tilting when a uniform downward pressure is applied, which can cause the blade to be oriented in a non- uniform angle or position relative to the seal for which the blade was designed. The blade may be at a poor optimal angle for cutting as pressure is applied with the cap tilted. The blade may slide laterally as pressure is applied with one hand and a bottle is held with another hand. The blade may be positioned or repositioned over the bottle rim where cutting of the seal will be ineffective.

Embodiments of the invention with an eccentric curved blade that extended beyond the edge of the cap, are less prone to tilting when a uniform downward pressure is applied because the rim of the cap will guide the cap and blade into the proper position as the rim surrounds the bottle mouth and forms a pilot tube. This prevents lateral movement with a narrow inner diameter the cap is less prone to tilting. The blade can be stabilized and reliably focused where and how it was intended to be used as pressure is applied and thereby improving the efficiency and ease of the puncturing and cutting process. If the blade extends beyond the threads but not beyond the cap rim, the cap rim could center the cap and position the blade. A downward force could cause the blade tip to make the initial puncture of seal bringing the threads of the cap closer to a point of making an overlapping engagement with the threads of the bottle neck, without the full interference of the seal. The cap rim can allow a positioned twisting motion of the blade to make a rotated cut in the seal in the plane of the bottle mouth and previously intact seal. As the rotational cut is made, the opening is made larger and eventually the thread end of the cap will be able to rotate to a position where they are able to match with the threaded end of the bottle neck and with the larger opening than the original puncture. The threads will be able to move to a position where the bottle neck threads overlaps and engages the cap threaded end. At this point, twisting the cap on the bottle with the threaded engagement will provide stabilization of the blade. Twisting will provide increasing shearing force and tension on the seal as the blade cuts through the seal along the leading edge of the blade. A result of using the curved blade and especially one that has a rotation of less than 360 through the pitch of the threads, is that the curved cut may make a curved incision that does not cut out a complete circle. Therefore, if the cap were to be removed a tab would be formed in the seal that could be used to pull the liner off the bottle mouth. In some cases, it will be desirable and necessary to completely remove the liner so that the liner does not interfere with the sealing of the cap on the bottle mouth. For example, the seal might add a height that interferes with the threaded engagement, or the seal might get folded over on itself causing a nonuniform surface height that may cause a bump or tunnel or gap that interferes with an effective seal of the cap. The surface of the seal may need to be removed for sterility reasons or it may need to be removed if fluid is to be poured out of the cap and the ruptured seal interferes with this process, for example by not having the widest opening possible for fast or precise pouring.

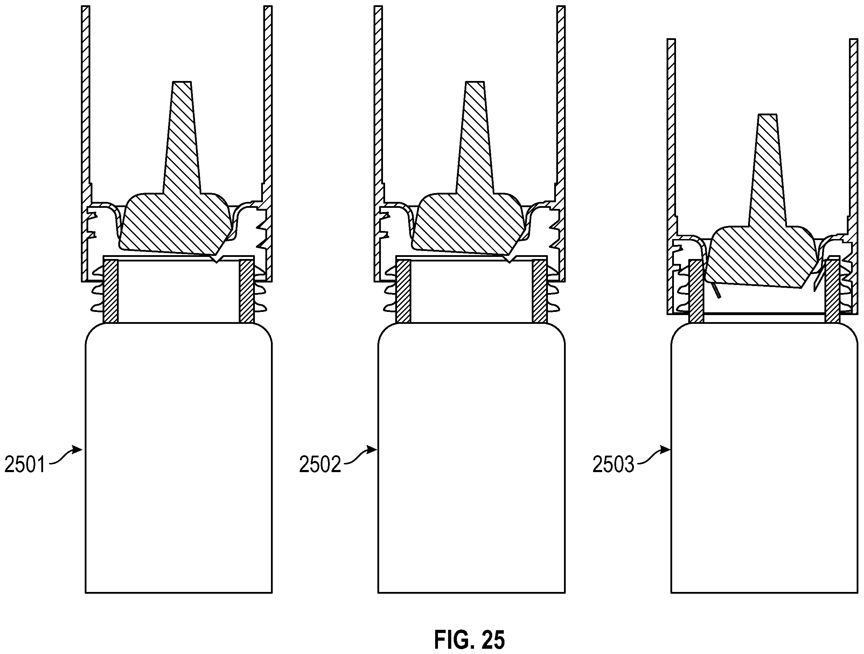

Alternatively, as shown in Figure 25, the blade tip may be recessed in the cap and within the threaded portion of the cap inner surface. In such a configuration the cap might be allowed to engage with the threads of the bottle in the alignment stage 2501, prior to puncturing of the seal in the a pressure stage 2502 leading to punctured stage 2503. This arrangement allows the blade to be stabilized throughout the entire cutting process and ensure that the blade is oriented in a specific cutting position with respect to the seal and the irrigation device. This configuration could also allow a cap with a cutter to be provided and stored in a pre-attached position with a secure threaded engagement. Further twisting off the cap could lead to puncturing of the seal by the user and activation of the device, such as by allowing fluid to flow through the central irrigation nozzle of a shielded irrigation cap.

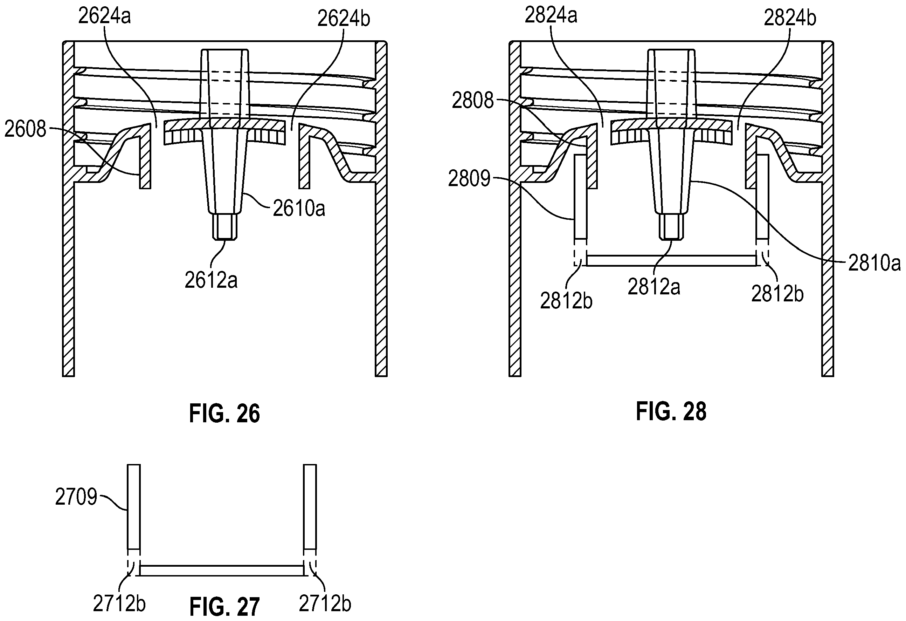

Further embodiments incorporate valves to alternate between direct and indirect flow of irrigation fluid through the device. For example, Figure 26 depicts an embodiment comprising inner wall 2608 (shown as a cross-section) extending from the partition between the fluid source connection end and the irrigation end of the device. The flow of the irrigation fluid passes directly through irrigation holes 2624a and 2624b toward the tissue to be irrigated, as useful for wound irrigation. Direct stream nozzle 2610a comprises a direct stream nozzle outlet 2612a.

Figure 27 provides a cap valve shown as a cross-section to illustrate the nozzle outlets 2712b that allow irrigation fluid to flow in an indirect stream from the device when cap valve is secured onto the inner wall 2608 by cap wall 2709. In certain embodiments, the cap valve may be secured by threadings present on inner wall 2608 and compatible threadings on cap wall 2709. Figure 28 illustrates the cap valve of Figure 27 secured to the irrigation device and thereby converting the device from an direct stream application as shown in Figure 26 to an indirect stream purpose. Irrigation fluid may flow directly through each of direct stream nozzle outlets 2824a,b and 2812a (after passing through nozzle 2810a). However, the cap deflects the stream into the irrigation end of the device through indirect nozzle outlets 2812b. The operation of securing and removing the cap to the inner wall 2808 and deflecting the direct stream may be understood to be a cap valve as referenced herein.

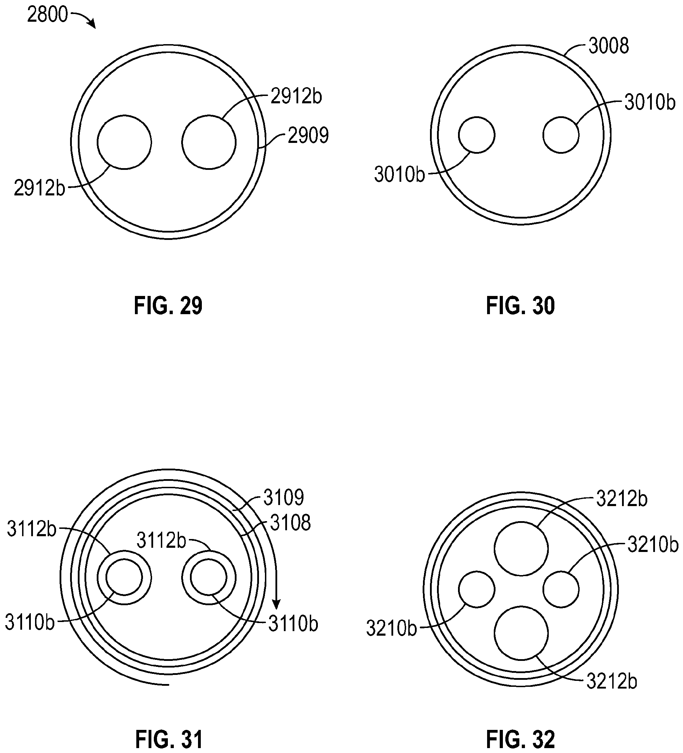

Further arrangements are also contemplated, for instance, where the cap secured may comprise holes to allow a direct stream to pass through when rotated into position, as shown by Figures 29-32. Cap 2900 of Figure 29 can comprise holes 2912b that are larger than the cross section of direct stream nozzle outlets 3010b such that when cap wall 2909 is seated onto inner wall 3008 the cap 2900 may be rotated about the inner wall 3010 to align the holes 2912b of the cap and the direct stream nozzle outlets 3010b, so that the irrigation fluid may pass from outlets 3010b and through the cap holes 2912b unobstructed. Figure 31 provides an example of this arrangement with cap wall 3109 secured to inner wall 3108 to achieve a direct stream with cap valve holes 3112b aligned with direct stream nozzle outlets 3110b. Figure 32 shows a closed position of the cap valve, where the cap valve holes 3212b are offset from alignment with direct stream nozzle outlets 3210b to achieve an indirect stream. The alignment in Figure 32 is achieved by a 90 degree rotation of the alignment shown in Figure 31, by rotation of the cap wall 3109 relative to the inner wall 3108.

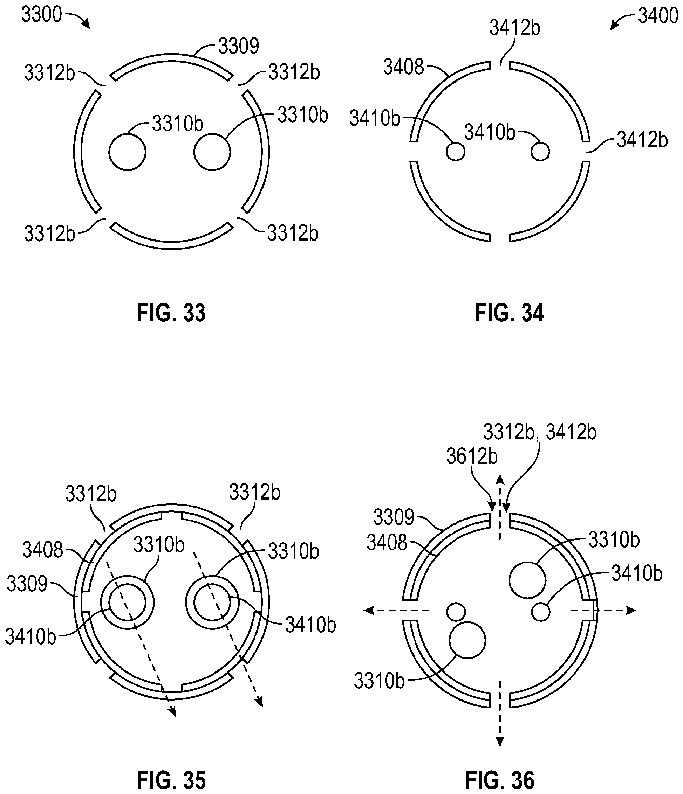

Further embodiments are depicted in Figures 33-36, where the cap and inner wall each can comprise perforations at their respective edges. As shown, cap valve 3300 can comprise direct stream cap holes 3310b which may be aligned with direct stream nozzle outlets 3410b when cap valve 3300 is secured by cap wall 3309 to inner wall 3408 of the irrigation device 3400. Note that irrigation device 3400 is shown in partial segment for clarity, and many features of this device can be common with other embodiments depicted throughout this disclosure. Cap 3300 can further comprise perforations 3312b within cap wall 3309 that may be aligned with corresponding perforations in inner wall 3408 and together, create an indirect stream nozzle outlet 3612b when aligned, as shown in Figure 36.

Figure 35 and Figure 36 show alternate arrangements of cap valve 3300 and inner wall 3408 of irrigation device 3400, demonstrating that rotation of the cap 3300 relative to inner wall 3408 alternate between an indirect stream with the perforations 3312b aligned with perforations 3412b and a direct stream where holes 3310b in the face of the cap are aligned with the direct stream nozzle outlets 3410b.

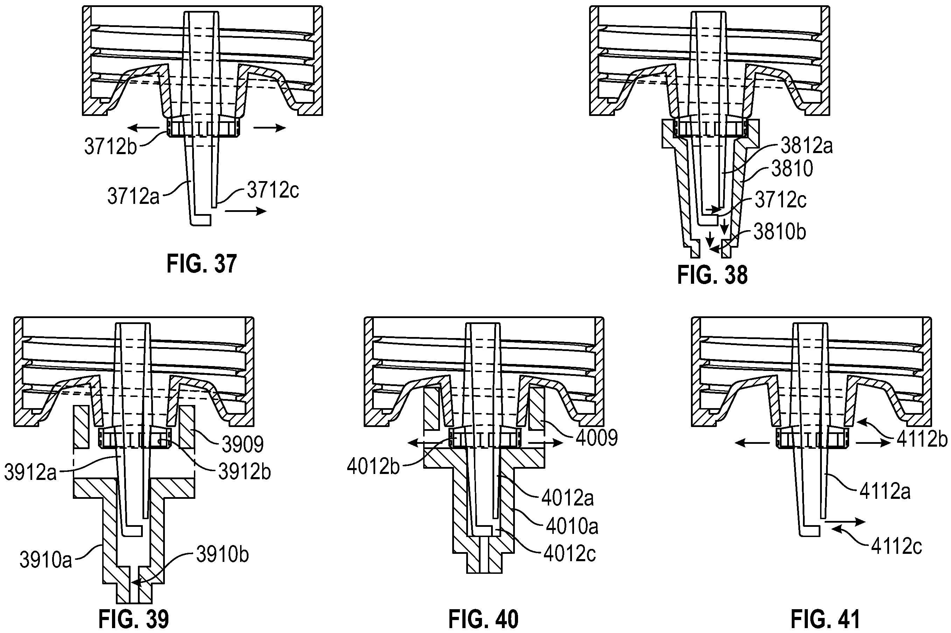

Further still, valves can comprise a cap secured directly to the nozzle, without need for an inner wall. As depicted in Figures 37-41, a nozzle can be configured with a lateral opening positioning the stream indirectly against the side wall (omitted from Fig. 37 for clarity) of the irrigation end of the device. The embodiment of Figure 37 has a central indirect stream nozzle 3712a with lateral facing ports 3712b about the medial portion of the nozzle 3712a, as in other embodiments described herein. However, the nozzle 3712a is terminated by a lateral-facing outlet 3712c, as opposed to a direct stream nozzle outlet. In this manner, the amount of flow throw the device may be increased and the ease of flow from the irrigation source to the irrigation end of the device may be improved. In addition, when used as an abscess irrigation device as shown, the irrigation fluid may be introduced at different depths, both within the abscess by inserting nozzle outlet 3712c beneath the surface of the skin, and by nozzle outlets 3712b at the surface.

Figure 38 shows the embodiment of Figure 37 with cap 3810 secured over nozzle 3712a. As shown, cap 3810 comprises a direct stream nozzle outlet 3810b, and changes the direction of flow from an indirect stream exiting nozzle outlet 3812c to a direct stream of flow. Thereby, the entire pressure of the irrigation fluid stream can be tailored for instance to the irrigation within an abscess.

Figure 39 shows a further embodiment, where a wall 3909 of the nozzle cap 3910a may be applied to the nozzle 3912a in an alternating position about the axis of nozzle 3912a to block the flow from nozzle outlets 3912b. Cap 3910a is shown secured to nozzle 3912a in an "out" position where the indirect stream nozzle outlets 3912b are blocked, allowing a direct stream for wound irrigation through nozzle outlet 3910b.

Alternatively, Figure 40 shows the cap valve 4010a in a "pushed in" position, exposing the laterally facing indirect stream nozzle outlets 4012b, and pressing closed the indirect stream nozzle outlet 4012c at the end of nozzle 4012a. In this manner, only the indirect stream nozzle outlets 4012b are opened. Cap 4010a may be removed completely, as shown in the embodiment of Figure 41, allowing irrigation fluid to stream through each of nozzle outlets 4112b and 4112c for a high volume indirect stream irrigation.

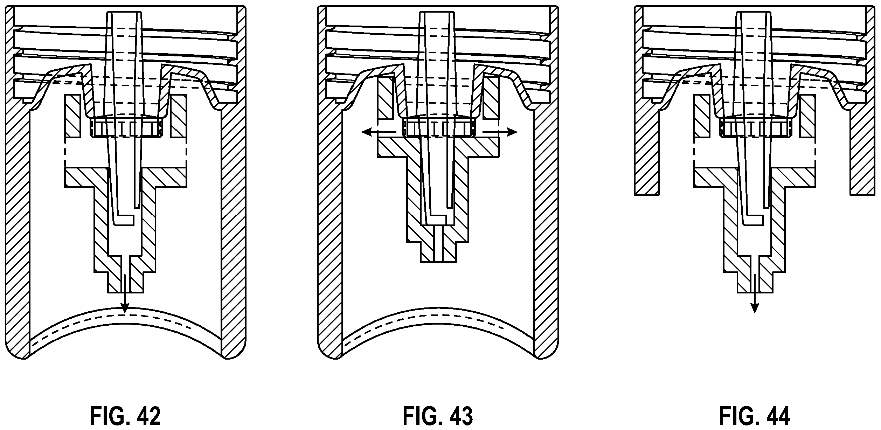

Each of Figures 39-41 are shown without the shield for clarity of the directional flow of each configuration, however, it will be understood by those of skill in the art that a shield may be provided as described throughout this disclosure to adopt the irrigation device to particular irrigation purposes. Figures 42-44 are provided to show how the shield may be included in each irrigation described for Figures 39-41 employing the cap valve to alternate indirect and direct irrigation streams. Note that removing the shield of Figure 44 allows the nozzle to be inserted into the abscess for irrigation at and below the surface of the skin without interference from the shield.

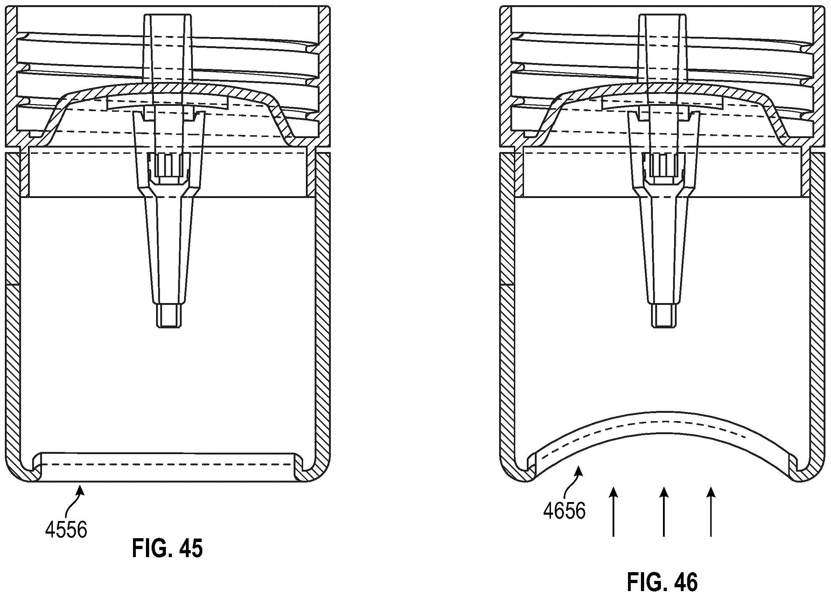

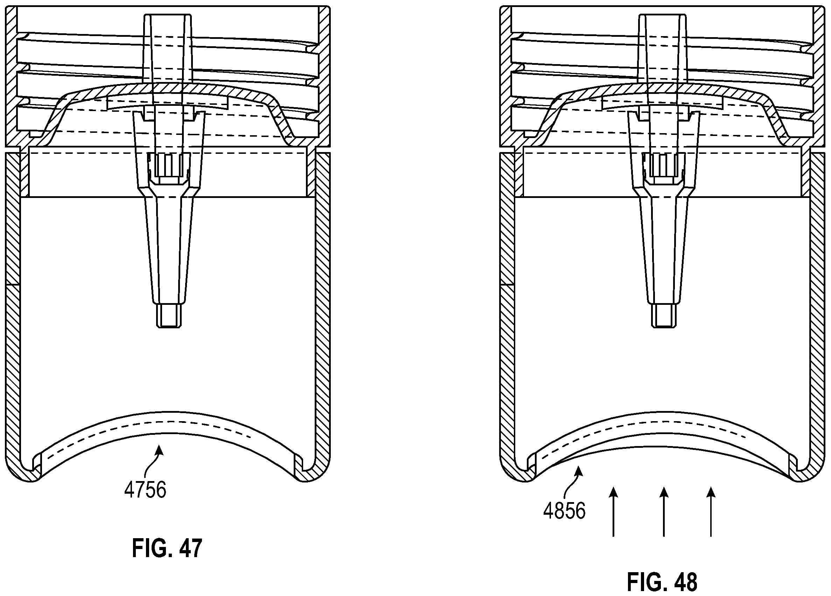

Figures 44-48 provide still further examples of the adaptability of irrigation devices disclosed herein, here concerned with the distal shield edge provided in a flexible manner. As shown in Figure 45, the flexible distal shield edge 4556 is provided to allow a straight edge for wound irrigation, but also adaptable to eye irrigation configuration as shown by shield edge 4656 in Figure 46 with arrows indicating an applied force at the distal shield edge. The flexibility of the distal shield edge 4656 is notable for combination with a rigid circumferential face, to allow the rigidity required to maintain the structural integrity of the device during use in either irrigation procedure. The devices shown in Figures 47 and 48 provide a further example where the flexible distal shield edge 4756 remains curved even when in a relaxed state, and is able to flex to a further curved position upon application of pressure, as shown by shield edge 4856.

Additional components of the irrigation devices disclosed herein can be described independently from the example embodiments provided by the figures and described above. Nozzles present in the irrigation devices disclosed herein (e.g., a first nozzle, a second nozzle, a nozzle comprising an indirect stream nozzle outlet) can comprise a nozzle inlet having any shape that allows irrigation fluid to flow into the conduit from the fluid source and/or fluid source connector, following connection of the fluid source to the fluid source connector. In certain aspects, the nozzle inlet can be simply an opening in the fluid source connector partition that allows fluid to enter. Alternatively, the nozzle inlet may extend within the fluid source connector. In either arrangement, the nozzle inlet can be compatible to accept a male Luer connector from the fluid source, and therefore in certain aspects can comprise a female Luer connector.

Where the fluid source comprises a male Luer connector, a syringe for instance, aspects where the nozzle inlet is a female Luer connector can allow direct connection of the fluid source to the first nozzle. Alternatively, or additionally, the fluid source connector can be configured to connect to a squeeze bottle by interaction between complementary threaded surfaces on the fluid source connector and the fluid source. As will be understood by those of skill in the art, and shown throughout the embodiments presented in the drawings, the fluid source connector can comprise a outer wall having an internally threaded surface. The dimensions of the outer wall and threaded surface are not limited to any particular size or shape and may be any that allow secure connection to external threaded surface of a fluid source, such as a squeeze bottle.

In certain aspects, the outer wall of the fluid source connector can extend from the partition to a proximal end of the fluid source connector. In certain aspects, the outer wall can comprise an internally threaded surface extends from the partition to the proximal end of the outer wall. In other aspects, the internally threaded surface extends from a distal end of the outer wall (e.g., the partition) to an intermediate point between the distal end of the outer wall and a proximal end of the outer wall. The intermediate point thus can define the point until which a threaded connection may advanced into the fluid source connector without rotation to reversibly join the respective threadings. The position of the intermediate point can vary in certain aspects and offer alternative advantages. In certain aspects, the threaded surface can extend to an intermediate point relatively close to distal end of the fluid source connector (e.g., the partition). In such aspects, intermediate point may be 20% of the distance between the partition and the fluid source connector, or within 0.5 cm. Such aspects, where the threaded surface terminates relatively close to the partition, may allow easier initial seating of the fluid source within the fluid source connector. After seating the threaded surface within the fluid source connector, the threadings may be advanced into the threaded surface of the outer wall to form a secure attachment.

Alternatively, the intermediate point may be relatively close to the proximal end of the fluid source connector, for instance 80 % of the distance between the partition and the fluid source connector, or 5cm. A proximal position may ensure the internally threaded surface has a sufficient length to allow an externally threaded surface of the fluid source to advance sufficiently to ensure a secure connection. In certain aspects, the intermediate point can be in a range from 20% to 80 %, from 25% to 75%, from 30% to 75%, from 40% to 70%, or from 50% to 65%, of the distance between the partition and the fluid source connector. In other aspects, the intermediate point can be in a range from 0.5 cm to 10 cm, from 0.5 cm to 5 cm, from 1 cm to 4 cm, from 1.5 cm to 4 cm, from 1.5 cm to 3.5 cm, or from 2 cm to 3 cm from the partition.

Those of skill in the art will understand that the outer wall therefore may have a non-threaded surface between the intermediate point and the proximal end of the outer wall. In such aspects the non-threaded surface may be smooth. The non-threaded surface of the outer wall may also be tapered or flared. Those of skill in the art will also recognize that in certain aspects, the outer wall may be cylindrical in part or in whole. For instance, the outer wall may be cylindrical at the threaded inner surface but flared between the intermediate point and the proximal end of the outer wall. Alternatively, the outer wall may be entirely cylindrical.

As stated above, in certain aspects the fluid source connector can comprise a partition that separates the fluid source connector from the remainder of the irrigation device. In certain aspects, the irrigation fluid can be delivered across the partition via a conduit in the first nozzle. In certain aspects, the partition can be planar. Alternatively, the partition can be curved, or partially curved. Where at least partially curved, the partition may be concave relative to the proximal end of the fluid source connector. Aspects where the partition comprises a concave curve may provide more volume in the irrigation cavity within the shield and also can allow a more proximal placement of the first and second nozzle. As shown in FIG. 2A, this may allow the first nozzle outlet to extend not beyond an abbreviated outer wall (e.g., a shield connector) of the device at the distal end of the fluid source connector.

The irrigation device may further comprise a shield connector. Generally, the shield connector may be any mechanism or structure to which a shield may be removably connected. In certain aspects, the shield connector can be a cylindrical outer wall extending distally from the fluid source connector, or more particularly from the partition within the fluid source connector.

### IRRIGATION METHODS

Irrigation methods employing the devices described herein are also made possible by the multifunctional arrangements, and contemplated herein. For instance, methods disclosed herein can comprise connecting an irrigation device to a squeeze bottle, performing a first irrigation procedure, removing the secondary nozzle from the irrigation device to expose a first nozzle, and performing a second irrigation procedure. In certain aspects, the first irrigation procedure can be a would irrigation performed by delivering irrigation fluid through a direct flow port in the secondary nozzle. In certain aspects the second irrigation can comprise an ocular irrigation. More commonly, it will be understood that a singular irrigation device as described above may be configured for any number of irrigation procedures, and thus the devices described herein independently applicable to ocular irrigation methods, wound irrigation methods, abscess irrigation methods, ear irrigation methods.

Methods for irrigating tissues using the devices described above are also contemplated herein. Particularly, irrigation devices described allow for the unique ability to perform multiple alternate irrigation procedures efficiently, without requiring multiple specialized irrigation devices for each procedure. As described above, the particular arrangement and characteristics of components within the irrigation devices disclosed above give rise to unique advantages that extend beyond those that may be generally expected from a multifunctional device.

For instance, in certain aspects, devices disclosed herein can be employed for abscess irrigation as a secondary function of the device. The device may be originally arranged wherein the second nozzle is a wound irrigation nozzle (e.g., a direct flow nozzle) and the first nozzle is a an indirect flow nozzle. In this manner, the device can be considered arranged in a wound irrigation configuration. However, a user could quickly adapt the device for abscess irrigation by removing the shield from the irrigation device to allow the first nozzle and second nozzle to extend beyond a distal edge of the device (e.g., the shield connector) such that the nozzles may be extended within the abscess. The second nozzle also may optionally be removed if a direct flow nozzle is not desired for irrigation of the abscess.

Alternatively, where devices comprise a saddle shaped shield, devices may be employed for ocular irrigation methods simply by removing the second nozzle comprising a direct flow outlet from the device, thereby exposing a first nozzle comprising an indirect flow outlet. Certain aspects can further comprise replacing a distal edge of the shield with an alternate shield edge suitable for ocular irrigation. The device may similarly be adapted to use in ear irrigation, without requiring the user to deglove, employ a new device, or otherwise interrupt or delay the irrigation method.

Of course, in certain aspects, the device also may be employed for wound irrigation methods. As stated above, U.S. 8,747,372 discloses wound irrigation devices and related methods. Wound irrigation procedures are routinely conducted in emergency settings for the removal of biologic infectious material from the wound, and also to periodically remove native immunologic debris from the wound during recovery. Despite a multifunctional ability, the wound irrigation methods disclosed herein may be conducted with every advantage of those previously disclosed.

Thus, wound irrigation methods employing the devices disclosed herein may be conducted by simply attaching the irrigation device to a fluid source, positioning the shield in contact with the skin, and flowing the irrigation source through the irrigation device. Because the device may come assembled with the second nozzle in a wound irrigation configuration (e.g., a straight flow tip directed at the wound), no further adaptation of the device is required prior to use. The irrigation device configured for wound irrigation by default allows the user to implement the device in its most commonly applied application.

In certain aspects, attaching the device to a fluid source can comprise forming a pressure connection between the fluid source and the device. The fluid source can be a syringe comprising a male Luer connector, and therefore may be attached by inserting the male Luer connector within a compatible female Luer connector of the irrigation device. Alternatively, the fluid source can be a squeeze bottle with externally threaded opening, and therefore the device may be attached to the fluid source by advancing the external threads of a fluid source into an internally threaded surface of the device.

## Claims

1. A multifunctional irrigation device comprising:
a fluid source connector configured to connect to an irrigation fluid source;
a first nozzle comprising a conduit which connects the fluid source connector and an irrigation end of the device in fluid communication;
optionally, a second nozzle;
a shield configured to be removably attached to the fluid source connector;
a direct stream nozzle outlet configured for direct irrigation; and
an indirect stream nozzle outlet configured for indirect irrigation.

2. The irrigation device of claim 1, wherein the first nozzle comprises each of the direct stream nozzle outlet and the indirect stream nozzle outlet.

3. The irrigation device of claim 1, wherein one of the first nozzle and second nozzle comprises the direct stream nozzle outlet, and the other of the first nozzle and second nozzle comprises the indirect stream nozzle outlet.

4. The irrigation device of claim 1, wherein:
the second nozzle is fixed to the fluid source connector;
the second nozzle comprises a conduit which extends through a partition of the fluid source connector and into an irrigation end of the connector; and
the second nozzle is fixed to the irrigation device.

5. The irrigation device of claim 1, wherein the second nozzle is removably secured to the first nozzle.

6. The irrigation device of claim 1, wherein the first nozzle terminates within the shield.

7. The irrigation device of claim 1, wherein the second nozzle terminates within the shield.

8. The irrigation device of claim 1, wherein the direct stream outlet is distal to the indirect stream nozzle.

9. The irrigation device of claim 1, wherein the fluid source connector further comprises a seal opener.

10. The irrigation device of claim 9, wherein the seal opener comprises a helical wall extending from a partition of the fluid source connector.

11. The irrigation device of claim 1, wherein the first nozzle comprises a valve to alternately direct a fluid stream through the first nozzle to either of the direct stream nozzle outlet or the indirect stream nozzle outlet.

12. The irrigation device of claim 11, wherein the valve rotates about a longitudinal axis of the first nozzle.

13. The irrigation device of claim 11, wherein the valve translates along a longitudinal axis of the first nozzle.

14. The irrigation device of claim 1, wherein a distal edge of the shield is interchangeable.

15. The irrigation device of claim 1, wherein a distal shield edge is flexible.
